# EUROPEAN PATENT SPECIFICATION

(11) **EP 1 694 386 B1**
(45) Date of publication and mention of the grant of the patent: **03.05.2017**
(21) Application number: 04813095.9
(22) Date of filing: 06.12.2004
(51) Int. Cl.: A61M 5/172, A61M 5/14

(54) **PATIENT-CONTROLLED ANALGESIA WITH PATIENT MONITORING SYSTEM**
PATIENTEN-KONTROLLIERTE ANALGESIE MIT PATIENTENÜBERWACHUNGSSYSTEM
ANALGESIE REGULEE PAR LE PATIENT AU MOYEN D'UN SYSTEME DE SURVEILLANCE DU PATIENT

(30) Priority: 05.12.2003 US 527197 P
(43) Date of publication of application: 30.08.2006
(73) Proprietor: CareFusion 303, Inc., San Diego, CA 92130 (US)
(72) Inventor: BOLLISH, Stephen, J., San Diego, CA 92129 (US); BROOK, Sandra, C., Solana Beach, CA 92075 (US); STEINHAUER, Thomas, C., San Diego, CA 92122 (US)
(74) Representative: Richards, John
(86) International application number: PCT/US2004/040718
(87) International publication number: WO 2005/056087

(56) References cited:
- WO-A-99/62403
- WO-A-03/053503
- US-A- 5 713 856

## Description

### Background

The present invention relates generally to patient care systems and more particularly, to a system and a method for controlling the self-administration of analgesics to a patient while monitoring a physiological parameter or parameters of the patient and providing information concerning such monitoring to prevent central nervous system and respiratory depression associated with administration of analgesics.

Programmable infusion systems are commonly used in the medical field to deliver a wide range of drugs and fluids to patients in a variety of settings. For example, syringe pumps, large volume pumps (herein referred to as "LVP"), and flow controllers are used in hospitals, clinics, and other clinical settings to deliver medical fluids such as parenteral fluids, antibiotics, chemotherapy agents, anesthetics, analgesics, sedatives, or other drugs. Single or multichannel systems are available, and different systems have various levels of sophistication, including automatic drug calculators, drug libraries, and complex delivery protocols. Still another type of drug delivery system is a patient controlled analgesia (herein "PCA") pump. With a PCA pump, the patient controls the administration of the narcotic analgesics since the patient is usually in the best position to determine the need for additional pain control. PCA is commonly administered via a stand-alone type of infusion device dedicated solely for PCA use. Examples of PCA devices are disclosed in U.S. Pat. No. 5,069,668 to Boydman and U.S. Pat. No. 5,232,448 to Zdeb.

Regardless of the type of pump system used, a serious side effect of the administration of drugs, particularly anesthetics, analgesics or sedatives, can be central nervous system and respiratory depression which can result in serious brain damage or death. For example, the infusion of anesthetics, analgesics, or sedatives using a syringe pump or LVP requires careful supervision by a trained medical professional to avoid overdosing. Even with infusion systems having sophisticated automatic programming and calculation features designed to minimize medication errors, it is not uncommon for patients to experience respiratory depression or other deleterious effects during the administration of narcotic analgesics or sedatives during in-patient or out-patient clinical procedures. Even in PCA applications, where overdoses are typically prevented by the patient falling asleep and therefore being unable to actuate a delivery button, there have been cases of respiratory and central nervous system depression and even death associated with the administration of PCA. The causes include clinical errors in programming the PCA device, errors in mixing or labeling analgesics, device malfunction, and even overzealous relatives who administer extra doses of analgesics by pressing the dose request cord for the patient.

Because of the potential dangers of narcotic analgesic overdose, narcotic antagonists such as naloxone (Narcan™) are widely available and commonly used in hospitals for reversal of respiratory and central nervous system depression. However, the effectiveness of such narcotic antagonists is highly dependent on prompt recognition and treatment of respiratory and central nervous system depression, as such depression can cause brain damage or even death due to lack of oxygen. Thus, respiratory and central nervous system depression must be recognized and treated promptly to assure a higher probability of successful recovery. Therefore, it would be desirable to monitor the actual physical condition of the patient to find respiratory or nervous system depression so that immediate remedial measures may be taken.

For the detection of potential respiratory depression associated with the administration of narcotic analgesics, sedatives, or anesthetics, a system that indicates a patient's respiratory status and cardiac status without the need to invasively measure or sample the patient's blood is particularly desirable and useful. Non-invasive end tidal carbon dioxide ("ETCO₂") and pulse oximetry monitoring are two such technologies used to monitor physiological parameters of a patient. The ETCO₂ method monitors the concentration of exhaled and inhaled CO₂, respiration rate, and apnea (respiration rate of zero) while pulse oximetry monitors the oxygen saturation of a patient's blood and the patient's pulse rate. The combination of ETCO₂ concentration, respiratory rate, and apnea or the combination of the blood oxygen saturation and pulse rate can be important indicators of overall patient respiratory and cardiac status. When using pulse oximetery to measure the blood-oxygen saturation, the term SpO₂ is commonly used and is used herein to indicate oxygen saturation.

One common approach to non-invasive pulse oximetry uses a dual-wavelength sensor placed across a section of venous tissue such as the patient's digit to measure the percentage of hemoglobin oxygenated in the arterial blood, and thereby measures the patient's oxygen saturation level. In addition, since the oxygenated hemoglobin at a specific tissue position is pulsatile in nature and synchronous with the overall circulatory system, the system indirectly measures the patient's pulse rate. Examples of similar pulse-oximetry sensors are disclosed in U.S. Pat. No. 5,437,275, to Amundsen et al., and U.S. Pat. No. 5,431,159, to Baker et al.

Another means of monitoring the respiratory status of a patient is by measuring and charting ETCO₂, a procedure known as capnography. In particular, current capnography devices utilize spectroscopy, for example infrared, mass, Raman, or photo-acoustic spectroscopy, to measure the concentration of CO₂ in air flowing through a non-invasive nose and/or mouthpiece fitted to the patient (e.g., ORIDION Corporation, http://oridion.com; NOVAMETRIX Medical Systems Inc., http://www.novametrix.com, and U.S. Patent Application Publication U.S. 2001/0031929 A1 to O'Toole). Capnographic ETCO₂ waveforms and indices such as end tidal CO₂ concentration, or the concentration of CO₂ just prior to inhaling, FICO₂, are currently used to monitor the status of patients in operating rooms and intensive care settings.

Patient care systems providing for central control of multiple pump units, potentially including PCA units, are known in the medical field. Examples of such systems are disclosed in U.S. Pat. No. 4,756,706 to Kerns et al., U.S. Pat. No. 4,898,578, to Rubalcaba, Jr., and U.S. Pat. No. 5,256,157, to Samiotes et al. Each of these prior art systems generally provides a controller which interfaces with a plurality of individual pumps to provide various control functions. An improved patient care system is disclosed in U.S. patent application no. 08/403,503 (U.S. Pat. No. 5,713,856) of Eggers et al. The central management unit of the Eggers et al. system can, for example, obtain infusion parameters for a particular infusion unit from the clinician and serve as an interface to establish the infusion rate and control infusion accordingly, individually control the internal setup and programming of each functional unit, and receive and display information from each functional unit. The Eggers et al. patient care system also provides for central control of various monitoring apparatus, such as pulse oximeters and heart monitors.

However, many prior systems described above do not provide integrated control of the PCA device in conjunction with a pulse oximeter and/or ETCO₂ monitor. Such systems would require constant dedicated monitoring by medical personnel in order for prompt detection and treatment of potential respiratory depression side effect associated with the administration of narcotic analgesics. Thus, these systems are not cost-effective because of the added expense from constant monitoring by medical personnel.

Furthermore, the systems discussed above do not automatically shut-off of the PCA unit in the event of respiratory depression. Without automatic PCA shut-off, these systems actually allow further administration of the narcotic analgesics which can further aggravate the respiratory depression until appropriate medical personnel arrives to intervene. The time for medical personnel to arrive and intervene will delay administration of narcotic antagonists and thereby potentially compromise their effectiveness.

Because of disadvantages associated with existing PCA systems, certain patients who might otherwise benefit from the PCA method of therapy may not be PCA candidates because of concerns about respiratory depression. Even if a patient were eligible for PCA treatment with prior art systems, these systems do not allow the patient to receive a more aggressive treatment because of the risk of inadvertent respiratory depression and thus the patient would not be able to obtain quicker and more effective pain relief from a more aggressive treatment.

In the more advanced systems that have provided substantial benefit to the art, such as that disclosed in US Patent No. 5,957,885 to Bollish et al. and US Pub. No. US 2003/0106553 to Vanderveen, control over PCA is provided in conjunction with monitoring a patient's physiological parameter or parameters. In the case of 5,957,885, an oximetry system is disclosed and in the case of 2003/0106553, a CO₂ system is provided. Both of these systems have provided a substantial improvement in the art. However, even further improvements are desired. For example, it would be a distinct advantage to provide a trend of respiration or heartbeat with the dosing of the analgesic superimposed so that a trend of the patient's physiological parameter and response can be seen clearly and rapidly. Additionally, expanding a drug library to specifically include various PCA dosing parameter limits would be of benefit.

WO 03/053503, corresponding to US 2003/0106553 to Vanderveen above, discloses a patient care system (90) comprising an interface unit (100), an infusion pump unit (150a), and a capnography unit (150b). The infusion pump unit (150a) is used to administer medical fluids to a patient, including the administration of anesthethics, analgesics, or sedatives. The capnography unit (150b) provides monitoring of the patient's expired air, in particular, the end tidal CO₂ levels in the expired air and the respiration rate. When the capnography unit (150b) indicates to the interface unit that the end tidal CO₂ level and/or pulse rate has reached a point outside a specified range, the interface unit (100) initiates visual and audio alarms and controls the infusion pump unit (150a) by modifying the flow or by shutting off the pump unit. In patient controlled analgesia applications, the interface unit (100) may block patient-controlled bolus doses of the analgesic until reset by a qualified medical professional or until the measured capnography values return to a normal range. The interface unit (100) also contains communication ports, which the interface unit can use to send signals to external devices, such as to alert medical personnel.

WO 99/62403 discloses a care system and associated methods for alleviating patient pain, anxiety and discomfort associated with medical or surgical procedures; the system comprising: at least one patient health monitor device (12a) coupled to a patient and generating a signal reflecting at least one physiological condition of the patient; a drug delivery controller (2a) supplying one or more drugs to the patient; a memory device (14a) storing a safety data set reflecting parameters of the at least one patient physiological condition; and an electronic controller (14) interconnected between the patient health monitor, the drug delivery controller and the safety data set; wherein said electronic controller manages the application of the drugs in accord with the safety data set.

Hence, those skilled in the art have recognized a need for a patient care system and method that can monitor the physical condition of a patient and can control the infusion of PCA to the patient based on the analysis. Further, those skilled in the art have recognized a need for a patient care system and method that can provide graphical information to a clinician to assist in determining the patient's condition and the response of the patient to doses of medical fluids so that remedial action may be taken as soon as possible, if necessary. The present invention fulfills these needs and others.

### Summary Of The Invention

The invention is defined by the system of independent claim 1. Preferred embodiments are defined by the dependent claims. Any disclosed methods are exemplary.

The present disclosure is directed to an apparatus and method for a patient care system comprising a pump for delivery of a medical fluid to a patient, a controller in communication with the pump for controlling operation of the pump, a monitor unit that monitors a physiological parameter of the patient and provides a measured value of a selected component of the a physiological parameter to the controller, and a memory with which the controller is connected, the memory comprising a stored range of acceptable values of the selected component of the physiological parameter, wherein the controller compares the measured value of the selected physiological component received from the monitor unit to the range of acceptable values for the component stored in the memory and if the measured value is outside the range stored in the memory, the controller performs a predetermined action, such as stopping the PCA infusion.

In another aspect, the monitor unit monitors the patient for a physiological parameter and provides a measured value of the physiological parameter to the controller. The controller automatically adjusts the rate of delivery of the medical fluid in accordance with the physiological parameter, and in a more detailed aspect, the controller automatically suspends delivery of the medical fluid by the pump to the patient if the measured value of the physiological parameter of the patient is outside the stored range of acceptable values.

In a further aspect in accordance with the invention, a graphical trend display is presented that includes a graphical display of the physiological parameter overlaid with an indication of the occurrence of a self-administered analgesic by the patient. In some level of detail, the display superimposes an indication of the time of occurrence of a self-administered analgesic over the waveform display of the physiological parameter of the patient. The physiological parameter may be waveforms of ETCO₂ or SpO₂ with an icon overlaying the waveforms representing the point at which a self administration of analgesic occurred. In another aspect, a tabular display is presented having relevant information to the PCA administration. In further detailed aspects, the tabular display includes the time of administration of the PCA and the levels of measured patient physiological parameters. In a more detailed aspect, the dose is also included in the tabular display.

In other aspects in accordance with the invention, the patient care system further comprises a PCA dose request switch with which the patient may request the pump to infuse a quantity of analgesic, wherein prior to allowing the pump to infuse the quantity of analgesic, the controller compares the measured value of the ETCO₂ received from the monitor unit to the range of acceptable values for ETCO₂ monitoring parameters stored in the memory. If the measured value is outside the range stored in the memory, the controller does not permit the pump to infuse the requested quantity of analgesic to the patient. In another aspect, a PCA dose request switch is provided with which the patient may request the pump to infuse a quantity of analgesic, wherein prior to allowing the pump to infuse the quantity of analgesic, the controller compares the rate of change of the ETCO₂ parameters received from the monitor unit to the range of acceptable values stored in the memory and does not permit the pump to infuse the requested quantity of analgesic to the patient if the rate of change is not consistent with the acceptable values. In yet further aspects, the controller also compares the respiration rate and apnea values of the patient to ranges of acceptable values and if outside those ranges, the controller does not permit the pump to infusion the requested quantity of medication to the patient.

In more detailed aspects, the patient care system further comprises a display on which is displayed a waveform of the physiological parameter of the patient as derived from a series of measured physiological parameter values provided by the monitor unit. Further, the monitor unit monitors the physiological parameter of the patient and provides a measured value of the physiological parameter to the controller. The controller automatically adjusts the rate of delivery of the medical fluid in accordance with the physiological parameter of the patient. In another aspect, the controller automatically suspends delivery of the medical fluid by the pump to the patient if the measured value of the physiological parameter of the patient is outside the stored range of acceptable values.

In more detailed aspects, the patient care system further comprises a display on which is displayed an ETCO₂ waveform of the patient as derived from a series of measured ETCO₂ values provided by the monitor unit. The shape of the displayed ETCO₂ waveform may be examined by a clinician to determine if a problem exists. A trend of waveforms may also be displayed and can be compared to one another so that a clinician may examine and compare multiple sequential waveforms to one another to determine if a problem exists. Further, the monitor unit monitors the expired air of the patient for ETCO₂ and provides a measured value of the ETCO₂ to the controller. The controller automatically adjusts the rate of delivery of the medical fluid in accordance with the end tidal CO₂ in the patient's expired air. In another aspect, the controller automatically suspends delivery of the medical fluid by the pump to the patient if the measured value of the end tidal CO₂ in the expired air of the patient is outside the stored range of acceptable values.

In yet further detail, the memory in which the range of acceptable values of the physiological parameter is stored is located at a position removed from the pump. In another aspect, the memory in which the range of acceptable values of the physiological parameter is stored is located in the pump.

In yet further aspects, the patient care system comprises an oximetry unit connected to the controller that monitors the blood of the patient and provides a measured value of the oxygen saturation of the patient's blood to the controller, wherein the memory comprises a stored range of acceptable values of the oxygen saturation of blood, wherein the controller compares the measured value of the oxygen saturation received from the oximetry unit to the range of acceptable values for the oxygen saturation stored in the memory and if the measured value is outside the range stored in the memory, the controller performs a predetermined action. In further detail, the controller automatically adjusts the rate of delivery of the medication in accordance with either of the ETCO₂ of the patient or the oxygen saturation of the patient's blood. In one aspect, this adjustment includes suspending delivery of the medication to the patient. In yet even further aspects, the oximetry unit also monitors the pulse rate of the patient and provides a measured value of the pulse rate to the controller, wherein the memory comprises a stored range of acceptable values of the pulse rate, wherein the controller compares the measured value of the pulse rate received from the oximetry unit to the range of acceptable values for the pulse rate stored in the memory and if the measured value is outside the range stored in the memory, the controller performs a predetermined action. In one aspect, this adjustment includes suspending delivery of the medication to the patient. Further, in another detailed aspect, the controller automatically adjusts the rate of delivery of the medication in accordance with any of the ETCO₂, FICO₂, respiration rate, apnea alarms, the oxygen saturation of the patient's blood, and/or the patient's pulse rate.

In yet a further aspect of the present invention, a patient monitoring system capable of providing communication and interaction between a PCA unit and a physiological parameter monitor is provided. In one aspect a pulse oximetry unit is used and in another detailed aspect, an EtCO₂ unit is used. The system would utilize signs of respiratory depression as recognized by one or more physiological values from the pulse oximeter unit and/or ETCO₂ unit and control the PCA unit accordingly. In one aspect, this control over the PCA unit includes suspending delivery of the medication to the patient.

In accordance with method aspects, there is disclosed a method for controlling patient self-administration of fluid infusion comprising monitoring a patient physiological parameter and providing patient physiological data concerning the monitored parameter. A processor compares the monitored physiological data to patient limits contained in a data base or library. A comparison signal indicative of said comparison is generated. The fluid infusion is terminated in response to a comparison signal representative of the monitored patient physiological condition being outside the patient condition limits.

In more detailed aspects, the patient care system further comprises a display on which is displayed an ETCO₂ waveform of the patient as derived from a series of measured ETCO₂ values provided by the monitor unit. Further, the monitor unit monitors the expired air of the patient for end tidal CO₂ and provides a measured value of the end tidal CO₂ to the controller. The controller automatically adjusts the rate of delivery of the medical fluid in accordance with the end tidal CO₂ in the patient's expired air. In another aspect, the controller automatically suspends delivery of the medical fluid by the pump to the patient if the measured value of the end tidal CO₂ in the expired air of the patient is outside the stored range of acceptable values.

In yet further apparatus aspects, there is provided an infusion pump for use with a container containing a given medication, said container including a machine readable label, the label specifying an identifier of the given medication and medication concentration and possibly other information about the given medication such as patient name, patient number, and patient location. The pump may comprise a pump mechanism which during operation causes the given medication to be delivered to a patient from the container, a programmable controller controlling the pump mechanism, a monitor unit that monitors a physiological parameter such as the expired air of a patient to measure a selected component of that air, and that provides a measured value representative of the measured component, a memory storing a drug library, said drug library containing a plurality of medication entries, there being associated with each medication entry a data set of associated delivery parameters for configuring the medication infusion pump, the memory also storing the selected component of the patient's expired air, there being associated with the selected component a range of acceptable values, a label reader which during use reads the contents of the label on the container, and means responsive to the label reader for identifying an entry in the drug library that corresponds to the given medication and configuring the programmable controller by using the set of medication delivery parameters associated with the identified entry from the drug library, wherein the programmable controller is configured to receive the measured value, compare the measured value to the range of acceptable values of the selected component, and to control the pump mechanism in accordance with the comparison.

In another aspect, in the case where an operator programs a medication delivery parameter into a medication infusion pump that is outside the acceptable range for the medication delivery parameter as contained in the data set of the drug library, the controller will provide a notice to the operator that the programmed parameter is outside the acceptable range for the parameter, and will also provide to the operator the actual limit or limits for the parameter that is in the drug library. This is, in effect, providing guidance to the operator as to what value to program for the parameter. Such medication delivery parameters and ranges include, but are not limited to, concentration limits, PCA dose limits, continuous infusion limits, loading does limits, bolus dose limits, lockout interval limits, and maximum cumulative limits. In another aspect, the guidance provided by the controller may not be a limit or limits of the data set but may be a value somewhere within the range. This may be considered to be a "preset" parameter value, or advisory parameter value, or other initial value. It can be programmed into the data set by the medical clinic, as can the ranges, medication names, and other information. Other information can include clinical advisories that are also included in the data set of the drug library. Such advisories provide notes to the clinician that are relevant to the medication being programmed for delivery to the patient.

In yet another more detailed aspect in accordance with the invention, a data set for medications used with PCA is created. The PCA data set includes parameters specifically regarding PCA, including but not limited to lockout interval.

In a further more detailed aspect, a controller may suspend, terminate, adjust, and restart PCA infusion based a value of a patient physiological parameters. In more detail, the above action may be taken when a value of one or more of the parameters of ETC02, FICO2, respiration rate, apnea, and pulse rate are outside predetermined range. Further, the lockout interval during which response to a patient's request for PCA delivery is suspended may be altered in response to a value of one or more of the above parameters.

In a further aspect, a monitoring device that monitors a patient physiological parameter or parameters module must be connected with the controller either directly or indirectly before an infusion can proceed.

Other features and advantages of the present invention will become more apparent from the following detailed description of the invention when taken in conjunction with the accompanying drawings.

### Brief Description of the Drawings

FIGURE 1 is a front view of an embodiment of a patient care system according to aspects of the present invention showing a large volume pump unit, a CO₂ monitoring unit, and a central interface unit interconnecting the large volume pump unit and the CO₂ monitoring unit;
FIG. 1a is an enlarged display of an ETCO₂ waveform trend presenting tabular and graphical information, with the ETCO₂ waveforms shown on time and pressure axes and the levels of the ETCO₂ and the respiration rate measurements presented in text alongside their respective acceptable ranges;
FIG. 2 is a front view of a patient care system according to further aspects in accordance with the invention showing a patient controlled analgesia pump, an ETCO₂ monitoring unit, and a central interface unit interconnecting the PCA pump and the ETCO₂ monitoring unit;
FIG. 3 is a back view of a central interface unit of the patient care system of FIGS. 1 and 2;
FIG. 4 is a block diagram of a central interface unit of the patient care system of FIG. 2;
FIG. 5 depicts an information display of the central interface unit of FIG. 2 during setup of a CO₂ monitoring unit showing areas for the input of values and showing a key for use in restoring values;
FIG. 6 depicts another information display of the central interface unit of FIG. 2 during setup of the CO₂ monitoring unit with certain values entered;
FIG. 7 depicts another information display of the central interface unit of FIG. 2 during setup of a PCA pump showing a selection of medication;
FIG. 8 depicts another information display of the central interface unit of FIG. 2 during setup of the PCA pump showing the unit selections made;
FIG. 9 depicts another information display of the central interface unit of FIG. 2 during setup of the PCA pump showing medication delivery values entered;
FIG. 10 depicts an information display of the central interface unit of FIG. 2 after completion of setup and during operation of the arrangement of FIG. 2;
FIG. 11 depicts an information display of the central interface unit of FIG. 2 with the patient care system in an alarm mode;
FIG. 12 is a front view of another embodiment of a patient care system in accordance with aspects of the present invention having a PCA pump, a CO₂ monitor unit, and a pulse oximeter monitor unit;
FIG. 13 is a front view of another embodiment of the patient care system in accordance with aspects of the present invention having a PCA pump and a combined CO₂/pulse oximeter monitor unit both of which are mounted to a central interface unit;
FIG. 14 depicts an information display of the central interface unit of FIG. 13 during setup of the SpO₂ pulse oximetry unit showing value fields;
FIG. 15 depicts another information display of the central interface unit of FIG. 13 during setup of the CO₂/pulse oximetry unit showing values entered in the fields to establish ranges of acceptable values of physiological parameters;
FIG. 16 is a block diagram of an infusion pump according to aspects of the present invention that includes an integrated CO₂ monitor and a pulse oximeter monitor in the same housing, both of which are tied to the controller of the pump;
FIG. 17 shows an information display by the central interface unit of FIG. 13 of a trend of ETCO₂ and respiration rate values with the points of boluses of PCA superimposed as crosses or plus signs;
FIG. 18 shows an information display by the central interface unit of FIG. 13 of trends of PCA doses and the patient's respiration rate;
FIG. 18a presents an information display by the central interface unit of FIG. 13 of trends of PCA doses and ETCO₂;
FIG. 18b presents a tabular information display by the central interface unit of FIG. 13 of the dose, ETCO₂, and respiration rate according to time;
FIG. 19 shows an information display by the central interface unit of FIG. 13 of a trend of SPO₂ and pulse values with the points of boluses of PCA superimposed as crosses or plus signs;
FIG. 20 shows an information display by the central interface unit of FIG. 13 of text indications of certain measured values, the acceptable ranges for those values, and a trend of the SpO₂ waveform;
FIG. 21 shows a sample drug library editor screen in which parameters concerning a drug may be entered into a data set fur use in a health care facility;
FIG. 21a shows a sample of a completed data set for a particular drug generated by a drug library editor program showing a medication name with associated medication delivery data, at which medications may be added to, edited, or removed from the library, delivery and other data may be associated with the medication names along with clinical advisories;
FIG. 22 presents a flow chart of a pump programming method in which the pump programming is compared to a drug library, trends are graphed, and reprogramming is attempted if the programming moves outside the drug library limits due to patient physiological changes; and
FIG. 23 is a clinical advisory included in a data set of a drug library that would be provided to a clinician who connects a PCA pump to the controller.

### Detailed Description of the Preferred Embodiments

The following documents are cited for reference: US Patent No. 5,957,885 to Bollish et al.; US Publication No. US 2003/0106553 A1 to Vanderveen; US Patent No. 5,681,285 to Ford et al.; and US Patent No. 5,713,856 to Eggers et al.

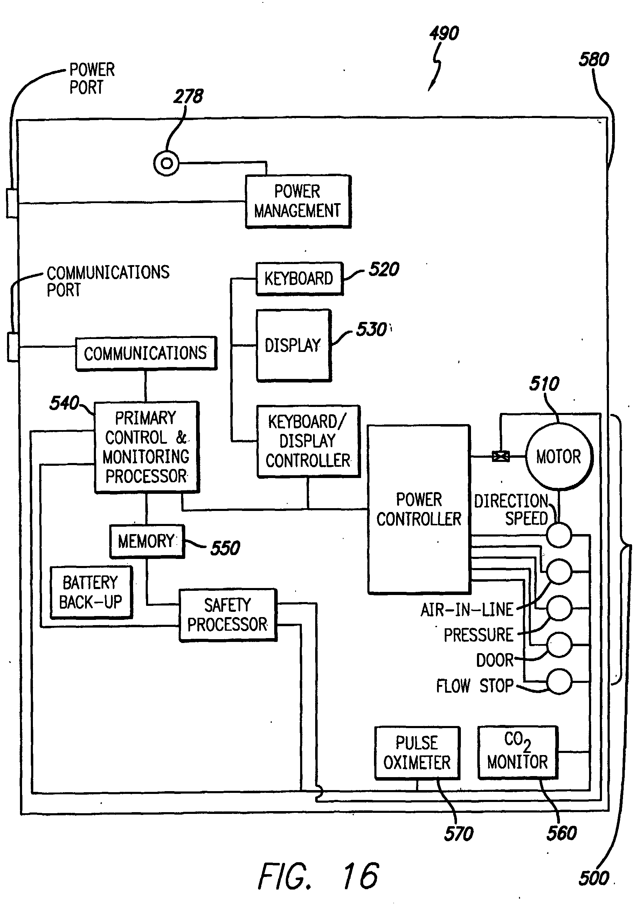

The following preferred embodiments of the present invention are described generally in the context of the programmable modular patient care systems disclosed in U.S. Pat. No. 5,713,856 to Eggers et al. filed Mar. 13, 1995 entitled "Modular Patient Care System," U.S. Pat. No. 5,957,885 to Bollish et al. filed Nov. 6, 1996 entitled "Oximetry Monitored, Patient Controlled Analgesia System," and US Publication No. US 2003/0106553 A1 to Vanderveen entitled "CO₂ Monitored Drug Infusion System." However, a person skilled in the art will recognize that the disclosed methods and apparatus are readily adaptable for broader application, including but not limited to other patient care systems and drug infusion pump systems. Moreover, as will also be appreciated by persons of ordinary skill in the art, any of an ETCO₂ monitored drug delivery system, SpO₂ monitored drug delivery system, and other systems, according to the present invention, can also be provided as stand alone integral units, as discussed in detail below and shown in FIG. 16.

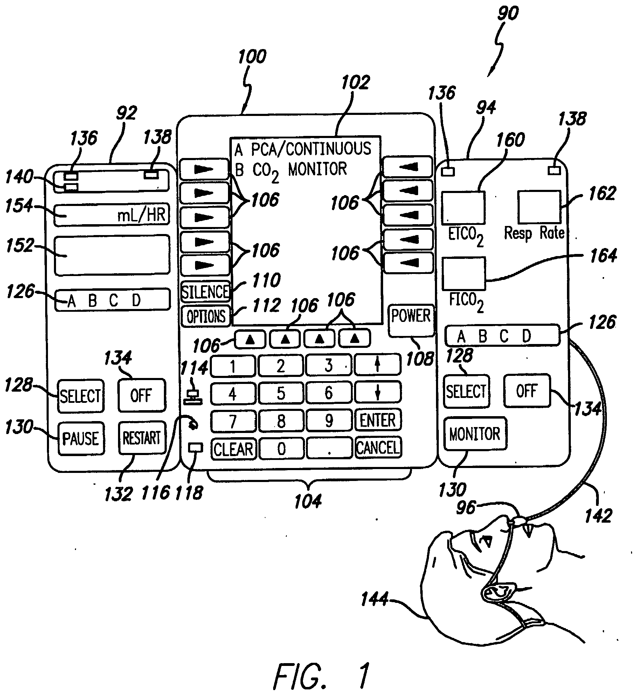

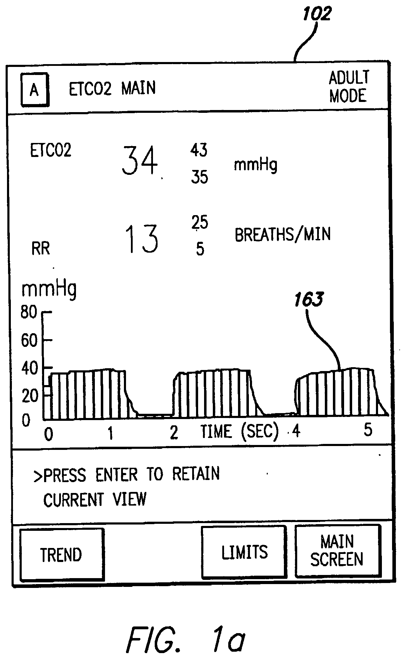

Referring now to the drawings with more particularity, in which like reference numerals among the several views indicate like or corresponding elements, FIG. 1 shows a front view of a modular, programmable patient care system 90 according to a preferred embodiment of the present invention. The patient care system 90 comprises a central interface unit 100, a PCA pump unit 92, a unit that monitors a patient's expired or inspired air 94 to determine the concentration of a selected component, such as a capnography unit, also known as an ETCO₂ unit, to measure ETCO₂, and an expired air sampling device 96 mounted to the patient. Although not shown, both the PCA pump unit and the ETCO₂ unit are connected to the patient. Although FIG. 1 shows only two functional units, *i.e*., the PCA pump unit 92 and the ETCO₂ monitoring unit 94, attached to the central interface unit 100, the patient care system 90 may additionally comprise other functional units, depending on a patient's particular needs. For example, one or more additional functional units can be connected to either the PCA pump unit or the ETCO2 unit, including but not limited to large volume pumps, flow controllers, syringe pumps, other air analysis monitors, pulse oximetry monitors, electrocardiographs, invasive and noninvasive blood pressure monitors, auditory evoked potential (AEP) monitors for monitoring the level of consciousness, cerebral blood flow monitors or cerebral oxygenation monitors, a thermometer unit, and others.

The central interface unit 100 generally performs five functions in the patient care system 90:
(1) it provides a physical attachment of the patient care system 90 to structures such as IV poles and bed rails;
(2) it provides a power supply to the patient care system 90;
(3) it provides an interface between the patient care system 90 and external devices;
(4) except for certain specific information, it provides a user interface with the patient care system 90; and
(5) it monitors and controls the overall operation of the patient care system 90,
including the integration of signals from monitor modules and/or pump modules in order to signal alerts and/or affect operation of one or more pump modules.

The central interface unit 100 contains an information display 102 that may be used during setup and operating procedures to facilitate data entry and editing. The information display 102 may also display various operating parameters during operation such as but not limited to the drug name, dose, infusion rate, infusion protocol information, patient lockout interval for PCA applications, ETCO₂ limits, FICO₂ limits, respiratory rate limits, apnea time period, and others. If other functional units are attached, such as a pulse oximeter, the information display 102 can display oxygen saturation, pulse rate limits, and/or other functional unit-specific information. The information display 102 is also used to display instructions, prompts, advisories, and alarm conditions to the user.

The central interface unit 100 also contains a plurality of hardkeys 104 for entering numerical data and, along with softkeys 106, for entering operational commands. In addition, the central interface unit 100 further contains a POWER hardkey 108 for turning electrical power on or off to the central interface unit, a SILENCE hardkey 110 for the temporary disablement of the audio functionality of the central interface unit, and an OPTIONS hardkey 112 for allowing user access to available system or functional unit options. The central interface unit may further contain an external computer indicator 114 for indicating that the patient care system 90 is communicating with a compatible external computer system, an external power indicator 116 to indicate that the central interface unit is connected to and operating with an external power source, and an internal power indicator 118 to indicate that the central interface unit is operating with the use of an internal power source such as a battery. The central interface unit may also include a tamper-resistant control function (not shown) which can lock out a predetermined set of controls. For example, once the infusion has been started, the central interface unit may not allow any changes to the infusion rate, or to other operation parameters unless an access code is first entered into the pump module or the central interface unit or a switch is actuated, such as a button located at the back of the pump module that is unlikely to be noticed except by clinicians. This assists in preventing infusion parameters from being changed by children or other unauthorized personnel.

The PCA pump unit 92 and the ETCO₂ unit 94 each include a channel position indicator 126 that illuminates one of the letters "A", "B", "C", or "D" to identify the channel position of that functional unit with respect to the patient care system 90. For example, the patient care system shown in FIG. 1 contains two channel positions A and B, with A to the immediate left of the central interface unit 100 (the PCA pump unit 92), and B to the immediate right of the central interface unit 100 (the ETCO₂ unit 94). Because both the PCA pump unit in channel A and the ETCO₂ unit in channel B are attached, as shown in FIG. 1, the information display 102 on the interface unit indicates A and B (note: in this embodiment, the PCA pump unit is designated on the information display as "PCA/Continuous" and the ETCO₂ unit is designated on the information display as "CO₂ MONITOR"). When the desired functional unit is selected by depressing the SELECT key 128 of a corresponding functional unit, the information display is configured so as to act as the user interface for the selected functional unit. Specifically, the information display is configured in accordance with a function-specific domain to provide function-specific displays and softkeys, as will become clear from the description of an example below.

Both functional units 92 and 94 of FIG. 1 have a SELECT key 128 for selection of the functional unit. The PCA pump 92 includes a PAUSE key 130 for pausing an infusion if the functional unit is a pump and if infusion is occurring. The ETCO₂ 94 unit includes a MONITOR key 131 for monitoring a function. The PCA pump unit also includes a RESTART key 132 for resuming a previously paused infusion subject to any access control features of the pump, as discussed above. Both modules also include an OFF key 134 for deselecting the channel, and if the functional unit on the channel was the only functional unit operating, for powering off the patient care system 90 at the same time. In addition, the PCA pump unit and the ETCO₂ unit each contain an alarm indicator 136 to indicate an alarm condition and a standby indicator 138 to indicate a standby condition. The PCA pump unit additionally contains an infusing indicator 140 to indicate an infusing condition. Each indicator illustratively illuminates when the respective functional unit is in the respective condition.

The PCA pump unit 92 contains a channel message display 152 that may be used to display informational, advisory, alarm or malfunction messages, and a rate display 154 that may be used to display, for example, the infusion rate at which the pump unit is operating. The PCA pump unit may also include a door with a door lock (not shown) within which the syringe or medication container is kept for providing security for narcotics or other medications to be infused. As known in the prior art, the pump unit can be a volumetric pump, a syringe-based pumping system, a parenteral type, or other appropriate configurations as can be readily determined by one skilled in the art. The PCA pump unit includes standard pumping and safety mechanisms to control various functions performed by the pumping device such as control of fluid delivery to the patient and monitoring of the fluid path for occlusion or air-in-line.

Connected to the ETCO₂ unit 94 and the patient is the air sampling device 96 which preferably collects air from the patient's nose and mouth and sometimes supplies oxygen to the patient. The expired air travels to the ETCO₂ unit through the line 142 where it is analyzed in real-time for ETCO₂ concentration by the ETCO₂ unit, preferably using infrared spectroscopy analysis. However, other ETCO₂ analysis techniques may be used as understood by persons of ordinary skill in the art. Alternatively, the sampling device 96 can include a sensor (not shown) for directly analyzing the expired air and sending a signal via the line 142 or via a wireless communication system (not shown) to the ETCO₂ monitor unit. The ETCO₂ unit includes several displays 160, 162, and 164 for displaying data to the user. For example, the ETCO₂ display 160 displays a numeric value for the ETCO₂ after expiration and before inhalation preferably in units of mm Hg or %. The respiration rate display 162 displays a rate value depicting the patient's current respiration rate, for example as determined by frequency analysis of the ETCO₂ waveforms. The display 164 presents the fractional inspired CO₂ or FICO₂ concentration in the patient's blood. A display of the ETCO2 waveform and other waveforms can be shown on the information display 102 of the central interface unit 100. Data shown in the waveform display preferably can be selectively extended or compressed for analysis of wave characteristics or for analysis of trends. The waveform data shown in the information display 102 may be smoothed, corrected, time averaged analyzed, or otherwise manipulated before display to provide optimal clinical value to the user. For example, the ETCO₂ unit could perform a running average to smooth the ETCO₂ waveform, and the horizontal time axis may be paused and/or adjusted for either ETCO₂ wave analysis or trend analysis.

As will be discussed in more detail below, data generated by the ETCO2 unit 94 is provided to the central interface unit 100, and may also be used to trigger an alarm, to signal an advisory on the information display 102, to automatically stop operation of the pump unit 92, or to otherwise adjust or control delivery of a drug or other medical fluid by the pump unit. For example, the interface unit is programmed in one embodiment to automatically stop the pump if the patient's ETCO₂ values fall outside a predetermined range of acceptable values. Alternatively, the pump and the monitor communicate directly with each other to affect delivery of fluid to this patient 144 based upon the monitored parameters. In yet another embodiment, the ETCO₂ monitor or interface unit includes a waveform analysis algorithm to analyze the ETCO₂ waveform and affect operation of the pump based upon certain waveform characteristics as are known in the art. In still another embodiment of the present invention, the interface unit includes a multi-parametric algorithm to calculate one or more indices of patient status using data from a number of different attached physiological monitors, and uses the calculated indices to affect control of the pump.

FIG. 1a is an example of an ETCO₂ waveform displayed on the information display 102 of the central interface unit 100. In this case, the monitoring module monitors ETCO₂ from the patient, processes the data, and presents certain data in textual form and in graphical form for presentation on the information display 102. In particular, the ETCO₂ of the patient is presented as the text number "34" in the measurement units of mmHg. The Respiration Rate ("RR") is also presented as a text number "13" measured in "breaths per minute." Additionally, a graph 163 is presented showing the ETCO₂ trend over time. In this case the time axis spans five seconds. Also shown in text form are the ranges of acceptable values for each parameter. In particular, the acceptable range for ETCO₂ is 35 to 43 mmHg. The acceptable range of values for the respiration rate is 5 to 25 breaths/min. While a graph for ETCO2 is shown in FIG. 1a, a graph of FICO₂ could also be selectable in another embodiment. Furthermore, respiration rate could be shown in graphical form as can apnea. If it is selected that FICO₂ is not to be graphed by itself, another embodiment is to indicate on the graph of ETCO₂, such as the one shown in FIG. 1a, where FICO₂ values are outside the acceptable range. Those unacceptable values could be displayed at the point on the ETCO₂ graph where the FICO₂ value was unacceptable, or could be displayed elsewhere.

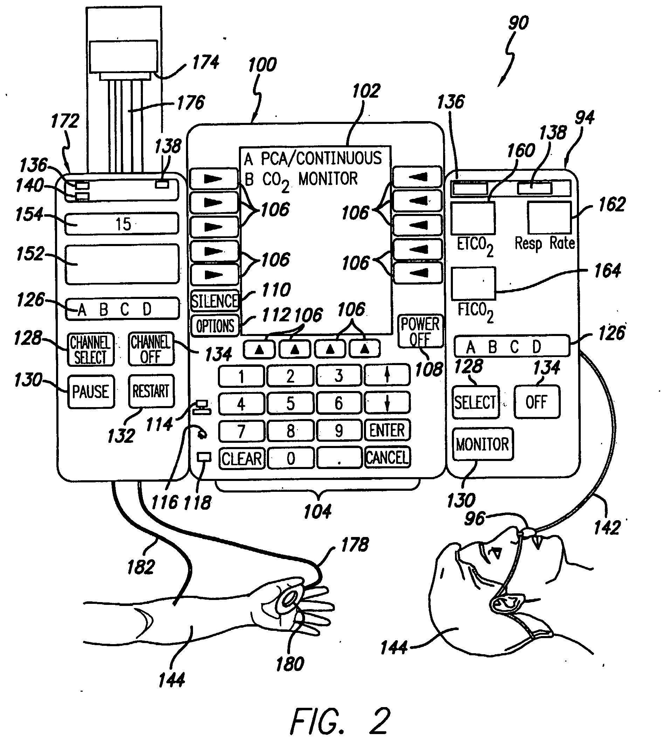

FIG. 2 shows an alternative embodiment of a patient care system 90, wherein the PCA pump unit 172 is a PCA syringe pump rather than an LVP pump. The PCA pump unit as shown has essentially the same interface displays and buttons as in FIG. 1; however, the PCA pump unit in FIG. 2 also includes a syringe pusher 174 and a syringe 176. The PCA pump unit further includes an infusion pumping device within its housing that drives the syringe pusher to infuse bolus doses of PCA drug from the syringe to the patient in response to commands from the central interface unit 100. The rate display 154 displays, for example, the infusion rate at which the PCA pump is operating or the patient lockout interval. The PCA pump includes a PCA patient dose request cord 178 connected to a handheld PCA dose request button 180 or other actuation device for patient use. The PCA drug is administered to the patient 144 through an IV administration line 182.

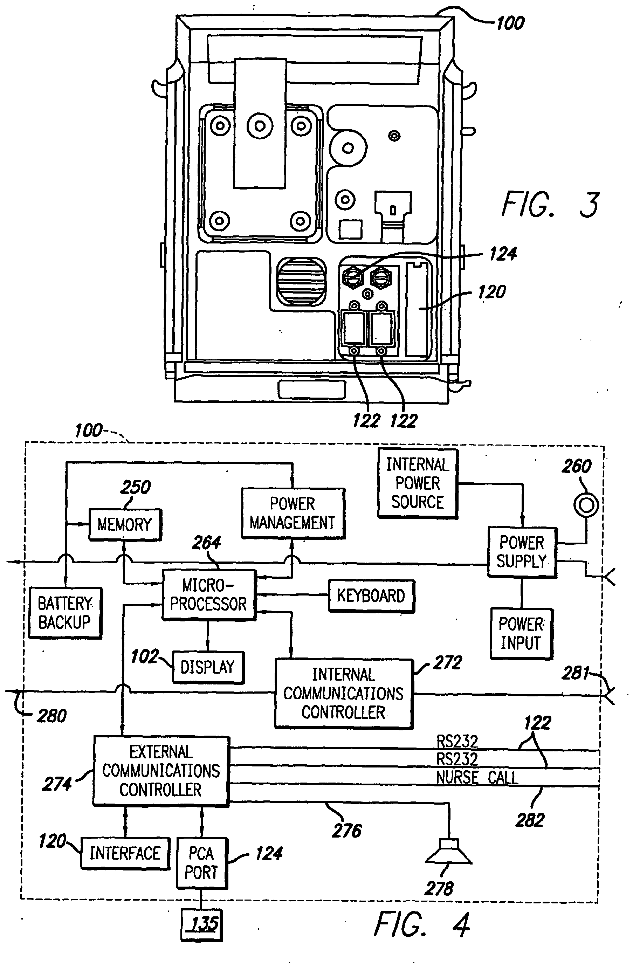

Referring now to FIG. 3, at the back of central interface unit 100 is at least one external communication interface 120, at least one interface port 122, and at least one PCA port 124 in this embodiment. The external communication interface 120 and the interface port 122 may be used to download and upload information and data and may also act as an interface-to-patient monitoring network and nurse call system, or as an interface to external equipment such as a barcode reader to provide a means of inputting drug and/or patient information from medication or patient records or from information and identification devices, such as barcodes, located on the patient, the nurse or clinician, on the bag of medical fluid, and other devices. Performing these functions with the external communication interface 120 and the interface ports 122 provide greater functionality and adaptability, cost savings, and reduction in input error. In particular, clinical errors associated with programming the pump unit 172 would be reduced, thereby reducing the risks of respiratory depression associated with the administration of sedatives, narcotic analgesics, anesthetics, or other drugs from use of the pump unit 172.

In this particular embodiment, the PCA port 124 provides a connection between the central interface unit 100 and one end of the PCA patient dose request cord 178 (cord shown in FIG. 2) if one of the mounted pump units is a PCA pump 172. At an opposite end of the PCA patient dose request cord is the hand-held dose request PCA button 180 or other PCA actuation device that can be actuated to request a dose of analgesic for the PCA patient. It is to be understood that although the central interface unit contains a PCA port 124 in this embodiment, in another embodiment such as that shown in FIG. 2, the pump unit 172 itself may contain a PCA port that would provide a similar connection from the pump unit, through a PCA patient dose request cord, to a dose request actuation device. Further, it should be understood that connectors may be placed elsewhere, such as on the front panels of the modules, bottom panels, or elsewhere.

Referring now to FIG. 4, which depicts a block diagram of a central interface unit 100 in accordance with aspects of the present invention, a microprocessor controller 264 receives and processes data and commands from the user and communicates with the functional units and other external devices. The microprocessor controller 264 directly controls the external communication controller 274 which controls the PCA port 123 and the data flow through the interface ports 122 and/or external communication interface 120. The microprocessor controller 264 also controls the internal communications controller 272 which controls the internal communication ports 280 and 281. The internal communication ports 280 and 281 are included in each functional unit as well as the central interface unit 100 and provide data and command interfaces between the central interface unit 100 and the attached functional units 150A, 150B.

During operation of the patient care system 90 such as the arrangement shown in FIG. 2, when the dose request PCA actuation device 180 is actuated, the microprocessor controller 264 receives the dose request signal via the patient dose request cord 178 and the PCA port 124. If the microprocessor controller 264 determines that there are no limitations in administering a requested bolus dose of narcotic analgesics, the microprocessor 264 would then send a signal to the PCA pump unit 172 via the internal communications controller 272 and the internal communication port 280 and/or the port 281, instructing the pump unit 172 to administer the requested bolus dose.

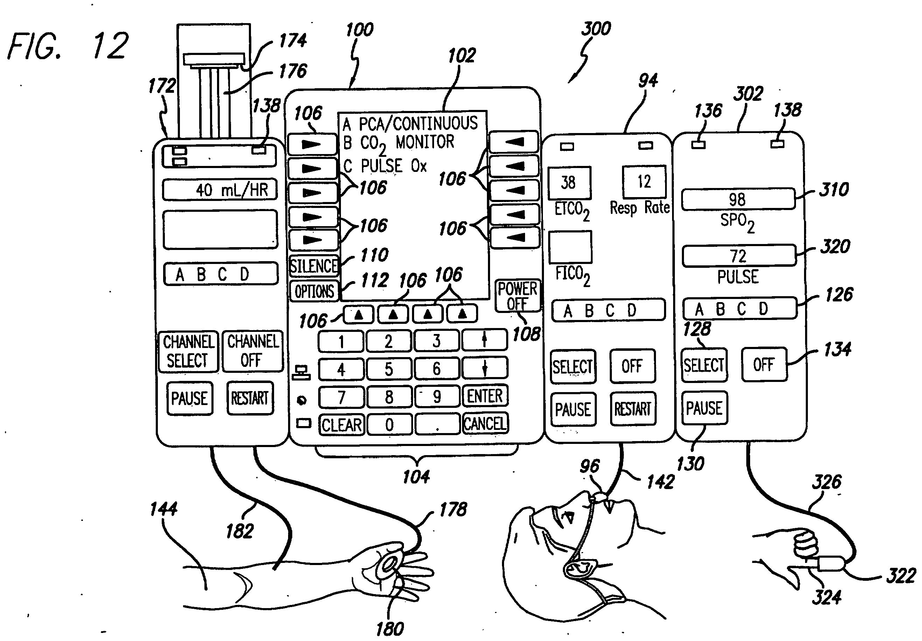

The microprocessor controller 264 also provides for the coordination of activities between the functional units, such as the PCA pump unit 172 and the ETCO₂ unit 94. For example, a clinician may set up the patient care system 90 with the PCA pump unit to provide PCA administration and the ETCO₂ unit to monitor the ETCO₂ parameters of a PCA patient. Optionally, one or more additional monitors, such as a pulse oximetry unit 302 as shown in FIG. 12, may be serially attached to the patient care system 90 and set up to monitor blood oxygen saturation and pulse rate, for example, as described in more detail below. The clinician may specify a minimum and/or maximum value for ETCO₂, respiration rate, and/or other monitored parameters which thereby effectively sets a range of acceptable values for those parameters. If the patient's ETCO₂ parameter is outside the selected acceptable range, such as in the case where it becomes less than the minimum or greater than the maximum levels set by the clinician, the ETCO₂ monitor 94 would send a trigger signal to the microprocessor controller 264 via the internal communications controller 272 and the internal communication port 280 and/or the port 281. In response, the microprocessor controller 264 may activate an audio alarm 276 to a speaker 278 as an example, send a visual alarm to the information display 102 (FIGS. 1 and 2), suspend operation of the PCA pump unit, adjust the flow rate of the PCA pump unit, and/or perform another predetermined function. For example, in response to an out-of-range ETCO₂ measurement in a PCA patient, the microprocessor controller 264 ceases all further administration of analgesics until after the unacceptably low or high ETCO₂ value and/or respiration rate situation is resolved, such as by clinician intervention or patient change. Alternatively, the microprocessor controller 264 may simply lock-out the PCA actuation device 180 so that the patient cannot obtain further self-administrations. Thus, after appropriate values have been set up, the central interface unit 100 provides communication and coordination between the PCA pump unit and the ETCO₂ unit 94 to ensure greater safety and decreased risk of injuries from respiratory depression.

In an alternative embodiment, rather than the microprocessor controller 264 suspending operation of the PCA pump unit 172 in response to only an out-of-range signal from the ETCO₂ unit 94 or from another functional module, the microprocessor controller would include program instructions for monitoring the changes in the CO₂ concentration data or other data generated by the ETCO₂ unit and to make decisions on whether to interfere with the patient's control of the pump module based upon the changes, such as the rate of change, in the monitored data.

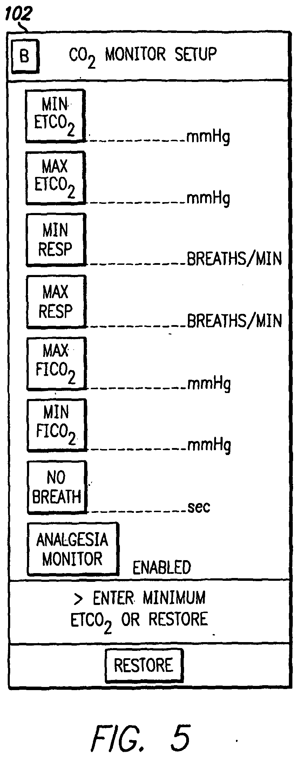

The interactions and functions of the central interface unit 100, the PCA pump unit 172, and the ETCO₂ unit 94 will now be described in conjunction with FIGS. 5-11 that show some of the step-by-step states of information display during the setup and operation of the patient care system 90. While the following example describes the setup of an operation of system 100 in a PCA setting utilizing a single PCA pump 172 and a single ETCO₂ monitor 94, one skilled in the art will appreciate that the present invention encompasses programmed infusion protocols utilizing other types and numbers of infusion pumps and monitors.

To set up a preferred embodiment of the patient care system 90, the clinician first attaches the air sampling device 96 to the patient as shown in FIGS. 1 and 2. The clinician then selects the ETCO₂ unit 94 and its corresponding channel by pressing the SELECT key 128 on the ETCO₂ unit (FIG. 2). By selecting the ETCO₂ unit, the information display 102 is configured so as to act as the user interface and thus provides ETCO₂ function specific displays as shown in FIG. 5. The information would be located on the display 102 (FIG. 1) such that selected information is adjacent softkeys 106 surrounding the display so that the operator may make selections and choices from the displayed information. The clinician can either input the minimum and maximum values by pressing the respective softkey and entering the associated limit numbers directly with the keypad 104, or by scrolling through numbers presented on the display by using the up and down arrows on the keypad and the ENTER key. More or fewer parameters may be included. In the embodiment shown in FIG. 5, ETCO₂, respiration, FICO₂ and NO BREATH values can be entered. However, in another embodiment, fewer parameters may be listed.

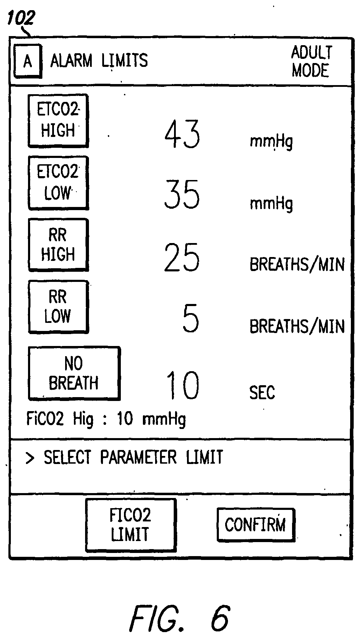

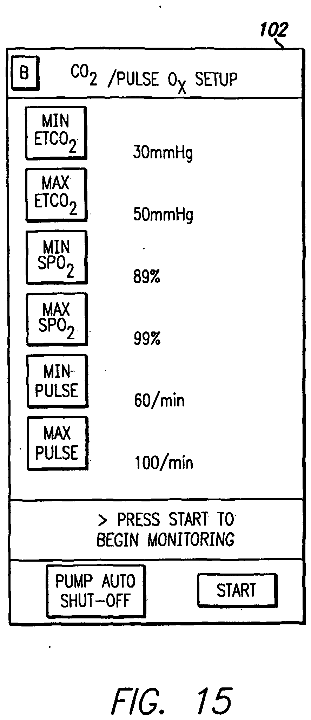

FIG. 6 shows the "ALARM LIMITS" information display 102 after the clinician has entered values or restored previous values. Prior to starting ETCO₂ monitoring by pressing the softkey associated with the START label (see FIG. 15 as an example of a START softkey), the clinician may select the PCA auto shut-off option for one or more other functional units, such as the PCA unit 172, so that the central interface unit 100 will shut off the selected functional unit(s) if the patient's ETCO₂, respiration rate, FICO₂, NO BREATH, or some combination thereof, falls outside of the specified maximum and minimum levels. Alternatively, the information display 102 could include parameters or selectable protocols for analyzing the patient's ETCO₂ waveform and setting limits on derived indices. Once ETCO₂ monitoring starts, the patient's ETCO₂ values, respiration rate, and ETCO₂ waveform are displayed in the central information display 102 as previously described and shown in FIGS. 1 and 2. Although the preferred embodiment of the patient care system 90 automatically initiates both audio 276/278 (FIG. 4) and visual alarms 102 for local alarm notification as well as notifies medical personnel remotely, such as by triggering a nurse call 282 if the patient's ETCO₂ or respiration rate falls above or below specified maximum or minimum levels, the patient care system 90 can be configured such that the clinician can also select specific alarms and notifications to medical personnel in such an event. An oximetry unit could also be used in place of an ETCO₂ unit or in addition to an ETCO₂ unit, as is discussed in more detail below.

In a preferred embodiment of the present invention, limit values for ETCO₂, respiration rate, and other parameters are stored in a data set in a memory 250 in the interface unit 100 (FIG. 4) or in the monitor 94 of the patient care system. In another embodiment, the data set can be stored elsewhere. Thus, rather than manually entering values using the numeric keys on the user interface 100 keypad 104 (FIG. 2), a user may recall pre-programmed values and/or configuration protocols from the stored data set to save time and minimize programming errors.

Storing a data set of institutional standards for drug infusion parameters and physiological parameter limits, such as the maximum and minimum concentrations of ETCO₂, FICO₂, the maximum and minimum values of respiration rate, and other values also aids in standardizing the quality of care in a clinical setting. In some embodiments, infusion parameter values or physiological parameter limits may be entered automatically from a machine-readable label, for example by using a bar code reader (not shown) with the barcode label mounted on the bag or on the syringe or other medical fluid container in which the medical fluid to be infused is stored. A radio frequency identification ("RFID") tag on the container may also be used and can be read by an RFID reader at the PCA pump unit 172 or at the user interface unit 100. Such infusion parameter values and physiological parameter values may also be entered by other means, such as through a connection with an external processor, such as a hospital server, through connection to a PDA, or other. Connections with these devices may be made in various ways, such as direct hardwired connection, infrared link, RF, use of an RFID chip with RF, a blue tooth link, or others.

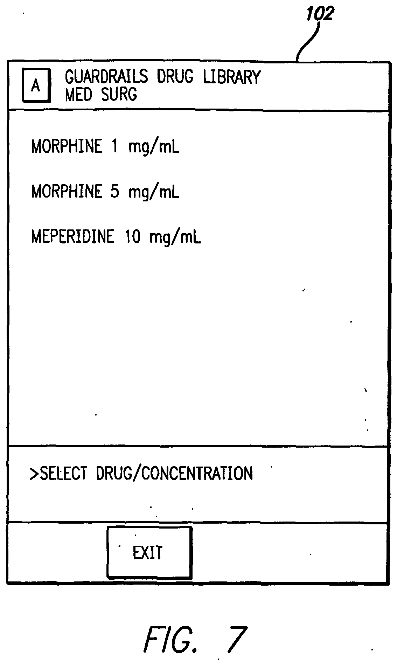

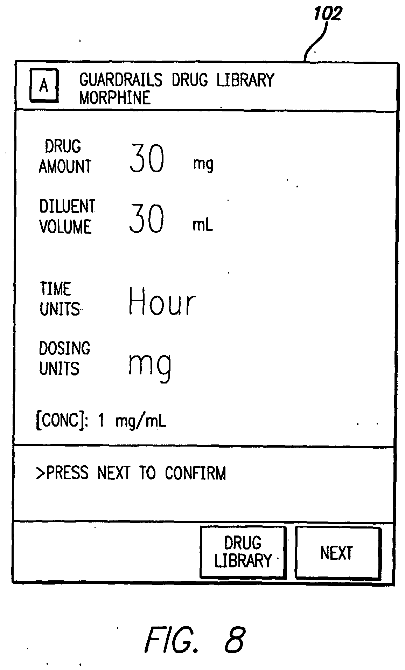

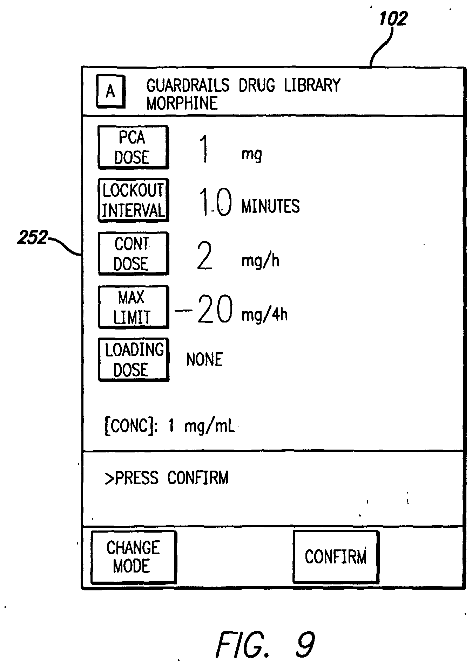

The clinician then selects the PCA unit 172 and its corresponding channel by depressing the SELECT key 128 on the PCA pump unit (FIG. 2). By selecting the PCA pump unit, the information display 102 is configured so as to act as the user interface and thus provides PCA pump function-specific displays and softkeys, as shown in FIGS. 7-9. In this example, the displays are PCA pump-specific. The clinician may first restore previous dosing units and the analgesic concentration or select the dosing units from, for example, mcg, mg, or ml, and input the analgesic concentration, as shown in FIGS. 7 and 8. Next, as shown in FIG. 9, the clinician may input or restore previous parameters for the patient bolus dose (PCA DOSE). For additional precaution to further prevent respiratory and central nervous system depression and as an alternative embodiment of the present invention, the patient care system 90 or the PCA pump unit may require the clinician to enter the patient request dosing limits, such as maximum dose per hour or per twenty-four hour period, or in this case, per four hour period ("20 mg/4h").

After entering the patient bolus dosage parameters and/or other drug delivery parameters, the clinician may choose to administer a background continuous infusion (CONT DOSE) of narcotic analgesics by pressing the softkey 106 adjacent the CONT DOSE label 252 (FIG. 9). Use of a background infusion in combination with patient-requested doses provides a level of narcotic analgesic sufficient for periods of low activity such as when the patient is sleeping. Thus, when the patient wakes up and requires additional analgesic because of increased activity levels, the patient can self-administer additional narcotic analgesics to meet those needs. If a background continuous infusion is selected by pressing the softkey 106 (FIG. 2) adjacent the CONT DOSE label 252 (FIG. 9), the information display 102 allows the clinician to input a desired continuous infusion dose. FIG. 9 shows the information display 102 after the clinician has entered values for both the patient bolus dose (PCA DOSE) and the continuous dose (CONT DOSE).

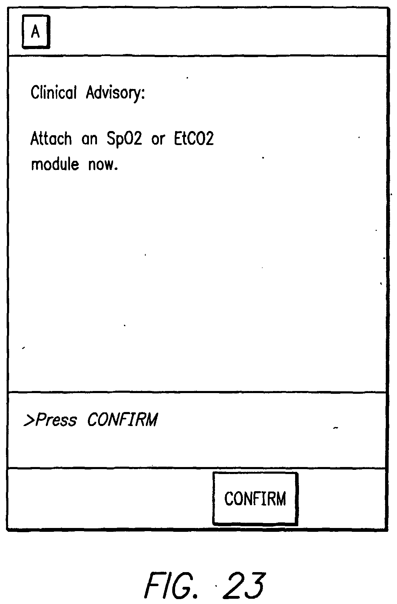

For parameters relevant to the PCA (pertaining to the infusion parameters shown in FIG. 9 and others), the following is a list of parameters that are included in a data set of a drug library, in one embodiment:
DRUG NAME - see the header of the screen of FIG. 9
CONCENTRATION - in units of mg/ml or other if desired, hard minimum and maximum (shown in FIG. 9 as "[Conc]")
DOSE LIMIT TYPE (for example, "soft" or "hard") (not shown)
MAXIMUM ACCUMULATED DOSE RANGE (for example, minimum over 2 hours and maximum over 2 hours). This is shown in FIG. 9 as MAX LIMIT.
PCA DOSE (minimum and maximum) *
LOCKOUT INTERVAL (minimum and maximum) *
CONTINUOUS DOSE rate - including units of ml/h so that drugs with just volumetric rates can be included, soft and hard minimums and maximums, and a default rate*
LOADING DOSE (minimum and maximum) *
BOLUS DOSE rate - include type such as disabled, hands-on, and hands-free; include dose units; include dose limits, soft minimum, soft maximum, and hard maximum;* a dose default; and a rate default
CLINICAL ADVISORY, ADVISORY NAME * (appears after drug selection and will be discussed in relation to FIG. 23)

A stored drug library may exist in the pump 172 or in the interface unit 100 or elsewhere that has preestablished values. These preestablished values may contain "hard" and "soft" limit values on dosing parameters and other medication delivery parameters. The limits may have been established by the clinic or institution within which the patient care system 90 resides. Also, for those parameters above with an asterisk (*), "preset" or "starting dose" values may be entered by the clinic or institution in the drug library data base or data set. When the operator indicates that such parameter is of interest, the preset will automatically be entered as the value, although the operator can change it, within limits established in the drug library.

Once the values have been entered into the patient care system 90 by the clinician as shown for example in FIG. 9, the microprocessor controller 264 according to its programming will enter a verification stage in which it compares each of these selected values against the stored drug library to verify that the selected values are within acceptable ranges. If a selected value contravenes a "hard" limit, the microprocessor controller will provide an alarm and require a value change before operation of the patient care system 90 can begin. If the selected value contravenes a "soft" limit, the microprocessor controller may require an acknowledgment from the clinician that he or she understands that the value entered is outside a soft limit and require an instruction that this value is nevertheless to be used.

Although in the presently preferred embodiment, the drug library is stored in the patient care system 90, the library or libraries may be located elsewhere. For example, in the case where the patient care system is connected to a hospital server or other server, such a drug library or data set or sets may be located at the remote server and the patient care system would communicate with the drug library stored in the remote server during the verification stage to obtain the acceptable ranges. As another example, the drug library may be located in a portable digital assistant (herein "PDA") such as a Palm Pilot ^{™}, or in a portable computer such as a laptop computer, or in a patient bedside computer, or nurse's station computer, or other location or device. Communications between the patient care system and the remote drug library may be effected by wired or wireless connection such as an infrared link, RF, blue tooth, or by other means. The clinician may carry the PDA having the drug library and before the patient care system will begin operation, it must communicate with the PDA to compare the hard and soft limits against the entered values. Other library storage and communications arrangements are possible.

Once the above steps have been completed, the clinician attaches the PCA administration set 182 (FIG. 2) to the patient's indwelling vascular access device (not shown) and presses the softkey 106 adjacent the START label on the central interface unit 100. The pump unit 172 is now operating with continuous monitoring by the ETCO₂ unit 94 of the patient's ETCO₂ parameters and/or with an SpO₂ unit 302 for the patient's oxygen saturation and pulse rate. The PCA pump unit begins background continuous infusion, if one has been programmed. In addition, the patient may now request a bolus dose of narcotic analgesics at any time by means of the patient dose request actuation device 180. Whether the patient actually receives a requested analgesic dose depends upon the patient request dosing limits, if any, as well as the patient's current ETCO₂ parameters relative to the limits set by the clinician.

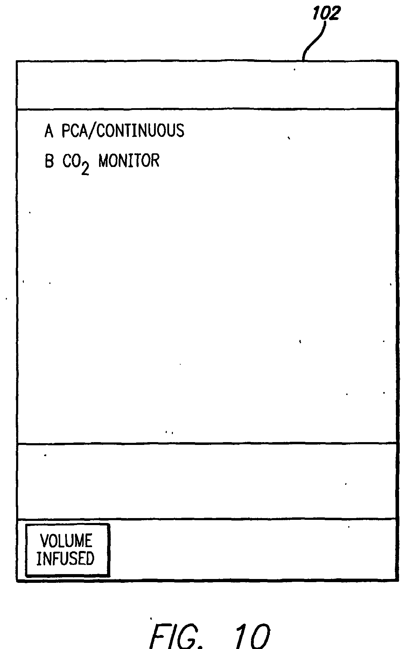

Referring now to FIG. 10, the positions A and B in the information display 102 of the central interface unit 100 advise the clinician which two functional units are located at channel positions A and B and that they are communicating with the central interface unit. The information display 102 may further be used to indicate the status of each functional unit occupying each respective channel in the patient care system 90. For example, the information display 102 at channel A, corresponding to the PCA unit 172 occupying channel A (FIG. 2), can be configured to indicate the patient bolus dosage and the background continuous infusion dosage. In addition, the information display 102 at channel B, corresponding to the ETCO₂ unit 94 occupying channel B, can be configured to indicate minimum and maximum ETCO₂ levels and respiration rates. The patient care system 90 may also be configured such that the information display 102 of the central interface unit 100 displays the patient's current ETCO₂ values and respiration rate. Naturally, if other monitors or pumps are attached, corresponding information from those units may also be selected and displayed on the central information display 102.

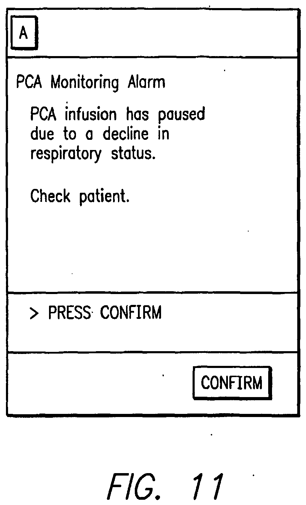

In the event that the patient's ETCO₂ parameters are outside the maximum and minimum levels set by the clinician, the central interface unit 100 immediately shuts-off or pauses the PCA pump unit 172, and thereby stops further administration of any background infusion and bolus doses. Optionally, the patient care system 90 may be programmed to adjust, rather than stop, the background continuous flow rate or bolus dose in response to ETCO₂ data or data received from other attached monitors, if any. As illustrated in FIG. 11, position A of the information display 102 indicates a "PCA Monitoring Alarm" status for the PCA pump unit. In addition, the central interface unit 100 activates an audio alarm 276 (FIG. 4) through a speaker 278 or otherwise, flashes the ALARM indicator 136 on the PCA pump unit 172 and/or ETCO₂ unit 94, and sends an emergency signal via the interface ports 122 and the external communications controller 274 in order to alert appropriate medical personnel, such as by a nurse call. Thus, faster response and intervention by medical personnel of the patient's respiratory depression from the administration of narcotic analgesics is provided.

Referring now to FIG. 12, an alternative embodiment of a patient care system 300 in accordance with aspects of the present invention includes the interface unit 100, the PCA pump unit 172, the ETCO₂ unit 94 as described above, and additionally includes a pulse oximetry unit 302 for providing the non-invasive measurement of blood oxygen saturation levels and pulse rate. The pulse oximetry unit 302 includes a pulse oximetry sensor 322, for example a dual wavelength sensor that attaches to a portion of the patient 144 containing venous flow, such as a finger 324 or earlobe. The pulse oximetry unit receives signals from the sensor through a connecting cable 326 and interprets the signals in accordance with the standard operation of a pulse oximeter as will be understood by persons of ordinary skill in the art. Examples of pulse oximetry sensors are disclosed in U.S. Pat. No. 5,437,275 to Amundsen et al. and U.S. Pat. No. 5,431,159 to Baker et al. From these sensor signals, the pulse oximetry unit can determine the patient's percentage of blood oxygen saturation, the SpO₂, and the pulse rate. The pulse oximetry unit contains an SpO₂ display 310 to display the patient's percentage of oxygen saturation and a pulse display 320 to display the patient's pulse rate.

A user may program the patient care system 300, for example using program steps similar to those described with reference to FIGS. 5-10, to signal an alarm, display an advisory, shut off the PCA pump unit 172, or alter operation of the pump unit if one or more of the ETCO₂, respiration rate, FICO₂, SpO₂, or pulse rate values, or some combination thereof, falls outside a selected range of acceptable values. In one embodiment, measurements from one or more of the functional monitor modules 94 or 302 may initiate a program sequence in the interface unit 100 that terminates a particular fluid delivery protocol and initiates a new delivery protocol from the PCA pump unit or another attached pump module (not shown) or simply terminates PCA pump operation.

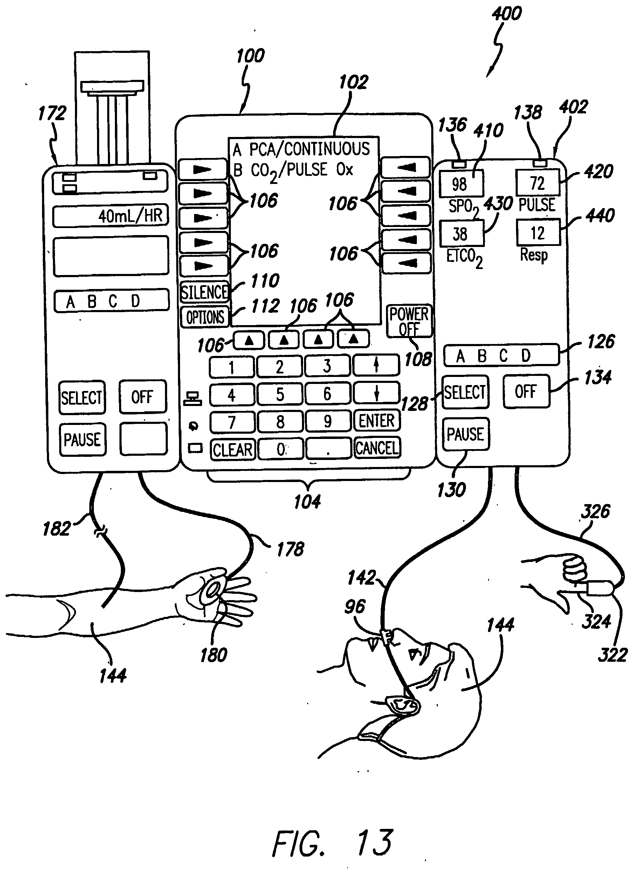

Referring now to FIG. 13, another embodiment of a patient care system 400 incorporating aspects of the present invention includes an integrated ETCO₂/pulse oximetry unit 402. The ETCO₂/pulse oximetry unit combines the functions of the ETCO₂ unit 94 (FIG. 1) and the pulse oximetry unit 302 (FIG. 12) as described above into one integrated functional unit. The ETCO₂/pulse oximetry unit 402 provides data to the central interface unit 100 for display on the information display 102 of SpO₂ 410, pulse 420, ETCO₂ 430, respiration rate 440, FICO₂ and other parameters, or fewer parameters and waveforms 450 for trending. The indicators 136, 138, and 126 and the switches 128, 130, and 134 are as described above with respect to other embodiments. The integrated ETCO₂/pulse oximetry unit can be programmed by the user, or alternatively by program information stored in the memory 250 (FIG. 4) of the interface unit 100 or in the ETCO₂/pulse oximetry unit itself. FIG. 13 shows a PCA pump unit 172 connected at the left side of an interface unit 100, and the combination ETCO₂ monitoring/pulse oximetry (SpO₂) unit 402 connected at the right side of the interface unit 100. Accordingly, the patient 144 has in his hand a PCA dose request button 180 connected to the PCA pump unit 172 through a cable 178 for controlling a bolus of analgesic to be administered to himself from the PCA pump unit through a fluid administration set 182. The patient is also monitored for his ETCO₂ level and respiration by an ETCO2 unit forming a part of the integrated unit 402. An expired air sampling device 96 is mounted in place at the patient's nose and mouth and communicates the expired air to the ETCO2 part of the integrated unit through the line 142. The patient is also monitored for blood oxygen saturation level with a pulse oximeter that forms a part of the integrated unit. A pulse oximetry sensor 322 is connected to the patient 324 and the sensor signals are communicated to the pulse oximetry portion of the integrated unit through the cable 326.

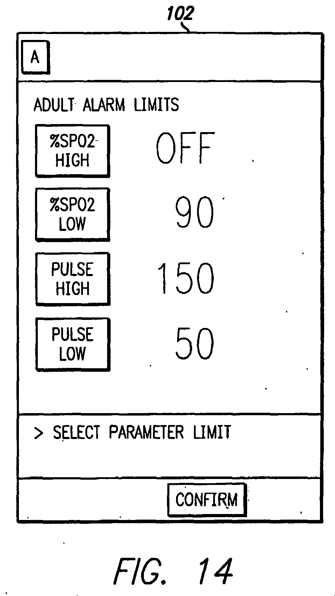

FIGS. 14 and 15 depict two examples of setup-screens displayed on the information display 102 of the central interface unit 100 directing the user to enter maximum and minimum values for each of the measured parameters and for initiating an infusion. In the case of FIG. 14, SpO₂ percentages and pulse rates for alarms are selectable. In the case of FIG. 15, ETCO₂, SpO₂, and pulse rate can be set on one screen. Respiration rate, apnea, FICO₂, and ETCO₂ can be set on another screen or in another embodiment; it may also be included on the same screen as shown in FIG. 15.

Referring to the block diagram of FIG. 16, an alternative embodiment of a patient care system 490 in accordance with aspects of the present invention comprises an integrated programmable PCA infusion pump 500 with a pump drive unit 510, a user interface for entering 520 and displaying 530 information, a microprocessor controller 540 that controls and monitors the operation of the user interface 520, 530 and the pump drive unit 510, and a memory 550 in communication with the microprocessor controller 540 for storing program instructions for operating the patient care system 490 and may also store a library or libraries for drugs, pumping parameters, and physiological parameters usable with monitors. The infusion pump 500 is generally similar to the infusion pump disclosed in U.S. Pat. No. 5,800,387 by Duffy et al., which is cited for reference. However, the patient care system 490 also includes an ETCO₂ unit 560 and a pulse oximeter unit 570 within the system housing 580. The microprocessor controller 540, like the central interface unit 100 of the above-described modular systems, monitors values generated by the ETCO₂ unit 560 and/or the pulse oximeter unit 570 and affects operation of the pump drive unit 510 in response to pre-determined changes in the measured values.

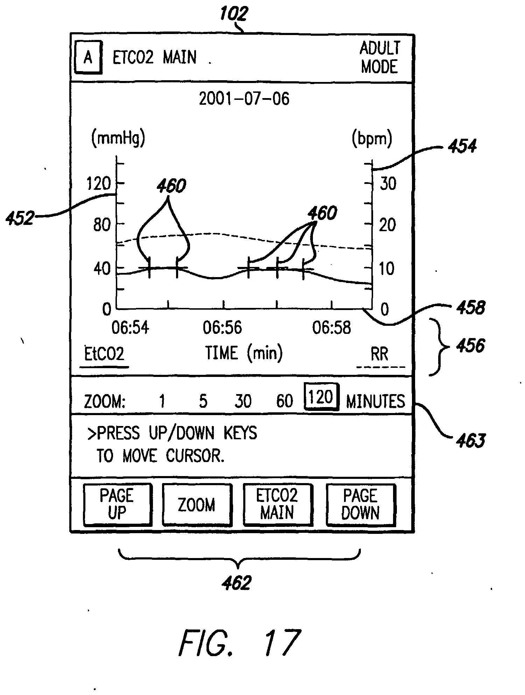

Turning now to FIG. 17, the first of a series of graphical displays is discussed. Such a display may be presented on the information display 102 of the central interface unit 100, or on another display device. In this case, a graphical presentation of data is occurring with two opposite Y axes, the left of which 452 is in the units of mmHg for the ETCO₂ measurement (pressure). The right Y axis 454 is in the units of breaths-per-minute for respiration rate. The legend 456 on the display indicates that the solid line is for ETCO₂ and the dashed line is for respiration rate. The X axis 458 is a time axis and in this case, spans approximately five minutes beginning at the time of 06:54. Also displayed is the application of a PCA bolus 460 shown as crosses or plus signs. With such a trend graph, effects of the PCA bolus can be more easily seen. For example, after the PCA bolus at approximately 06:55, the patient's ETCO₂ pressure decreased by approximately 10 mmHg but recovered in approximately one minute. The display also includes certain softkeys, 462 such as ZOOM, PAGE UP, ETCO₂ MAIN, and PAGE DOWN. The ZOOM feature includes a plurality of selectable periods of time 463 so that trends over longer or shorter periods may be quickly selected and studied.

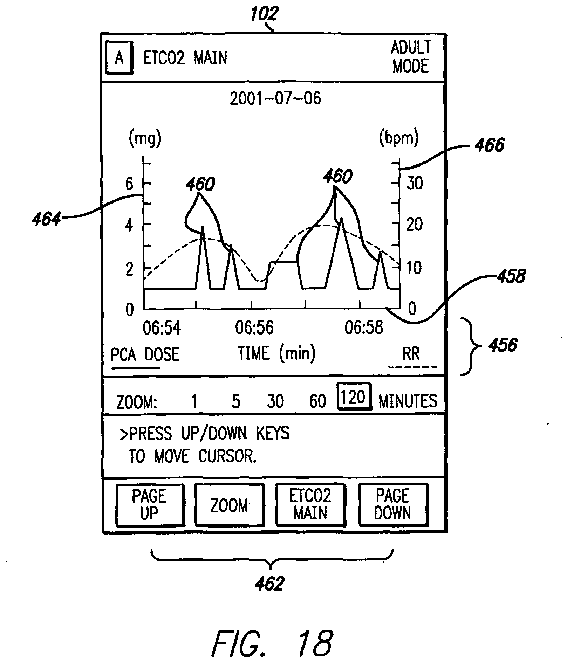

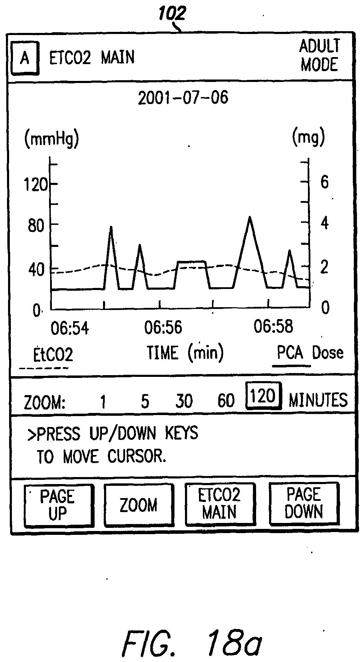

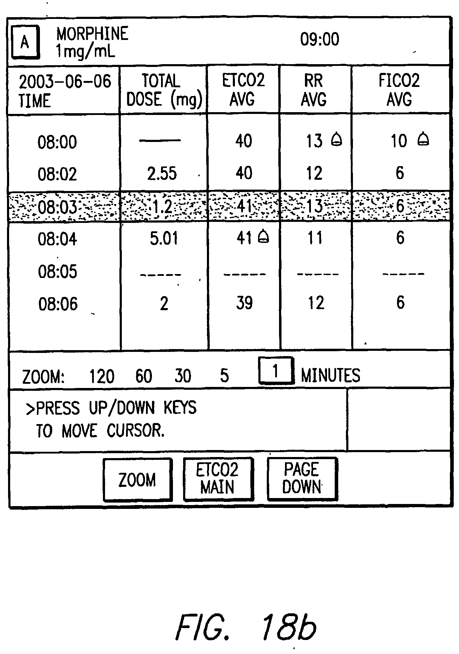

FIG. 18 is similar to FIG. 17 in that an ETCO₂ graph is provided as a display on the display 102 of the central interface unit 100. However in this figure, the respiration is plotted against the quantity of the PCA dose. The left Y axis 464 provides the quantity of PCA dose in milligrams (mg) while the right Y axis 466 provides the respiration rate in breaths per minute. The X axis 458 is in units of time, in this case the same approximately five minutes time period as shown in FIG. 17. A continuous dose of approximately one mg is being provided while the PCA doses are recognizable from the peaks above the continuous dose level. The PCA doses are in solid lines while the respiration rate is in dashed lines. A dip in the respiration rate occurs just after the time of 06:56 following two PCA doses occurring within the same minute. The first dose is approximately 4 mg and the second is approximately 3 mg. Even though the patient again infuses a PCA dose at the time of 06:56, it is a lower level dose, approximately 2 mg, spread over a longer time period and the patient's respiration rate recovers. However, after another two spiked doses at approximately the time of 06:58, one of which if approximately 4 mg and the second of about 2.5 mg, the respiration rate begins to decrease again at the time of 06:59. The same softkeys 462 are available as in FIG. 17.

FIG. 18a shows a trend of the patient's ETCO₂ over time with an opposing Y axis of dose in mg. Thus, the effect of the PCA doses on the patient's ETCO₂ can be seen with the trend graph of the ETCO₂ which is superimposed on the same chart showing the trend of PCA doses.

Another array of data for the central interface unit 100 information display 102 is shown in FIG. 18b. In this case, tabular data concerning dose, ETCO₂, respiration rate, and FICO₂ are given. The data is organized by time, which is placed in the left column. In that case, the tabular data is organized by the time frame of 08:00 through 08:06.

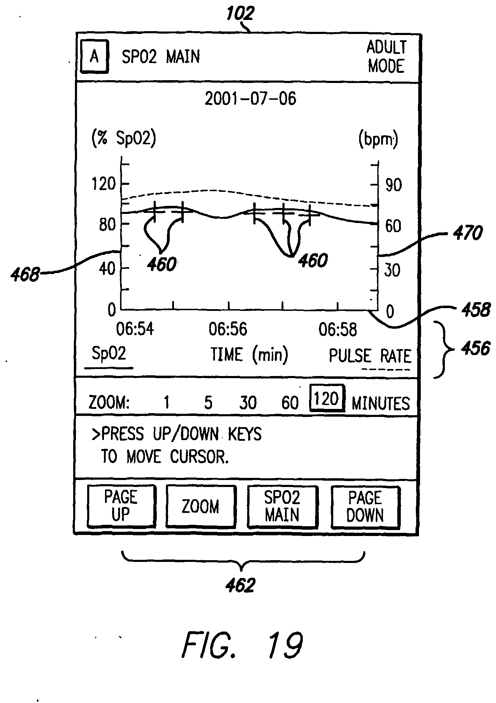

FIG. 19 is directed to oxygen saturation and in this case, is a pulse oximetry graph on the central display 102. In this display, there are provided a curve of SpO₂ in solid lines and the patient's pulse rate in dashed lines. The left Y axis 468 is in percentage of oxygen saturation while the right Y axis 470 is in the units of beats per minute (pulse rate). The X axis 458 is the same five minutes as in FIGS. 17 and 18 and the PCA doses are shown as crosses. Once again, the trends can be seen due to the graphical nature of the display. For example, two PCA doses occurred at about 06:54 and immediately after, the patient's oxygen saturation went from approximately 95% to 80% within about one minute. The patient's pulse rate at that same time period increased from approximately 80 bpm to 90 bpm. Both the oxygen saturation and the pulse rate began to recover within one minute but then the patient self administered another three PCA doses with similar effects in oxygen saturation and pulse rate. Similar softkeys 462 are available as in the other displays in FIGS. 16, 17, and 18.

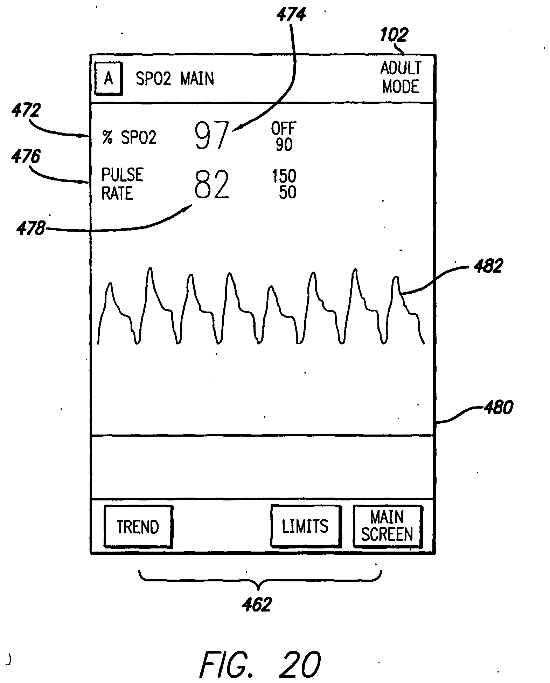

FIG. 20 shows in text the ranges for SpO₂ 472 and the current percentage reading 474. In this case, the acceptable range that has been programmed is 90% to no upper limit. Also shown in text is the range 476 for the pulse rate, i.e., 50 to 150 beats per minute or bpm and the current reading of 82 bpm 478.

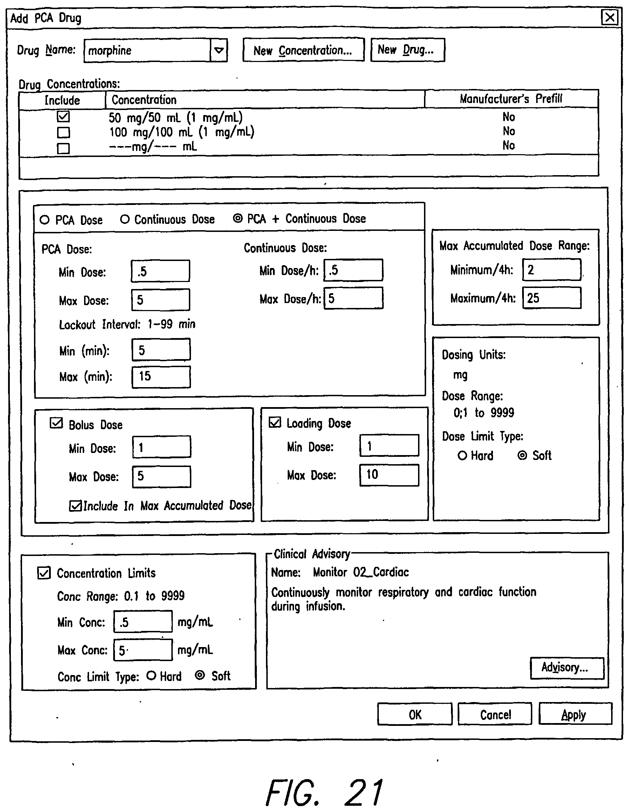

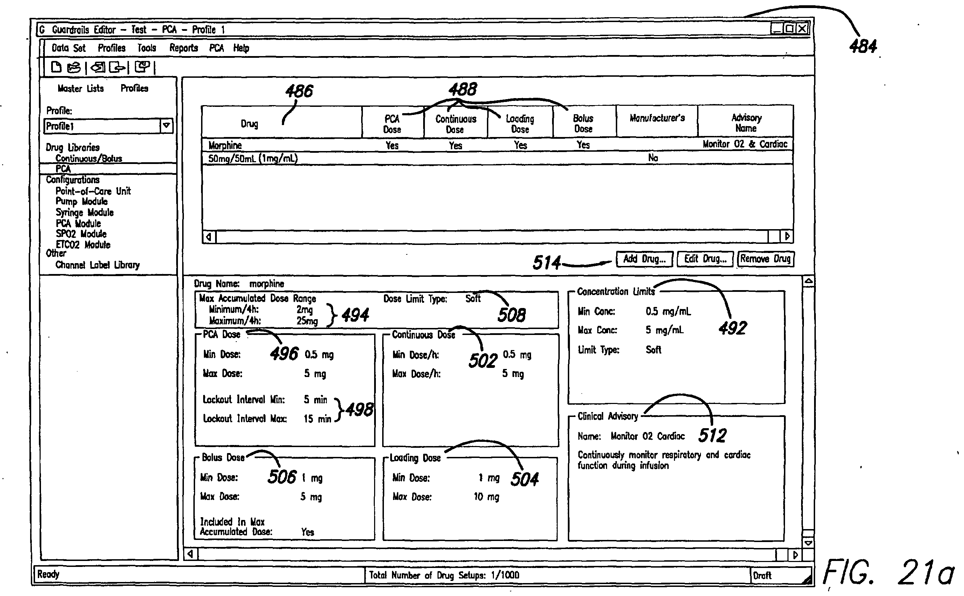

Referring now to FIGS. 21 and 21a, the operation of a drug library editor program can be seen. In FIG. 21, there is shown a data screen 483 in which data concerning a particular drug, morphine in this case, can be entered to form a part of a data set. This screen is used to build a data set around the identified drug for the drug library. Choices are provided at the top of the form 483 for "New Concentration ..." or "New Drug ..." It will be noted that the first box vertically below the drug name identification concerns concentration. Selecting "New Concentration ..." above would involve this box. On the other hand, selecting "New Drug ..." would involve the selection of a different drug to build a data set around. Proceeding downward, the PCA dose type can be selected. In this case, selections include "PCA Dose," "Continuous Dose," and "PCA + Continuous Dose." Other dose-specific information can be entered in other boxes, such as "Bolus Dose," "Loading Dose," "Max Accumulated Dose Range". The dosing units of mg are indicated at the right side of the screen. At the bottom left of the screen, the ability to enter "Concentration Limits" is provided and they may be specified. Clinical advisories may be entered at the bottom right, and one can be seen in this example. A user of this Drug Editor program can generate and edit an extensive library of medications that may be infused into a patient or otherwise delivered to a patient. That library can include not only an extensive list of medication names, but the user of the program can also generate and edit a wide array of data concerning each of those medications.

After the data set for morphine has been established through an editor program such as that described in conjunction with FIG. 21, a specification for the established data set of the particular drug may be examined. An example of such a specification is shown in FIG. 21a. In particular, a screen 484 containing the data set information about the medical "drug" morphine is shown.

Further data regarding the medications included in the drug library can also be associated with "patient-specific" data such as the physiological monitoring that is performed in accordance with some aspects discussed above. For example, the drug library may include an alternate maximum dose for a medication that is higher or lower in accordance with the measured ETCO₂ of that patient, or in accordance with the measured SpO₂ of that patient, or in accordance with other measured physiological conditions of the patient. The data may also include an indication that the medication is entirely unsuitable for a patient having a certain physiological measurement. Such a data set about a drug may also require the clinician to connect a physiological monitor to a patient before infusion can begin. In the example of a PCA application, an ETCO₂ monitor may be required by the data set before infusion can begin. Such a monitoring requirement can be entered into the data set for the particular drug in one embodiment.

Additional "patient-specific" data can be included in a drug library. For example, another field in the data base for each medication may be an "allergy" field. In such a field, an allergy code or name may be entered. Should a patient have such an allergy, the data for that medication may include different limits for the administration of the medication or administration of any of the particular medication to such an allergic patient may be prohibited. Data concerning a patient's past medications may become relevant when the drug library includes such data related to its medication entries. For example, a medication entry in the drug library may specify that the medication is only to be delivered at a lowered maximum dose to a patient who recently received another particular medication within the last twelve hours. The patient's history of medication deliveries at the health care facility would be considered in the case of such a comprehensive drug library. The method of delivery of the prior medications would not be relevant, only the fact that the patient received the medication.

The drug library also contains "soft" limits and "hard" limits. A "soft" limit for a medication is a maximum and/or minimum outside of which administration of the medication is permitted but is questioned since it may be higher or lower than the standard practice. An example of a "soft" limit is an infusion rate that is higher than standard practice but is not so high as to cause permanent injury when the length of the infusion is controlled. A "hard" limit on the other hand is a maximum and/or minimum outside of which administration of the medication is prohibited. An example of a "hard" limit is an infusion rate that is so high that permanent injury is likely. Another example for a "hard" limit is the case where a patient's measured ETCO₂ is at such a depressed value that the administration of any dose of a particular medication could cause permanent injury. In such a case the "hard" limit for the particular medication is zero and any attempted administration of the medication will be prohibited. Thus, the library has additional data entries corresponding to physiological measurements of the patient. Other library data may include a field or fields for soft minimum and maximum limits on patient weight, an alarm limit for pump occlusion pressure, and volumetric infusion rates (ml/h), for example, a hard maximum on a continuous rate, and a hard maximum for a bolus rate. The library may also include a syringe list to allow brands/models to be enabled/disabled to minimize the chance of inadvertently selecting the wrong type on the pump.

Returning now to FIG. 21a, a medication data screen 484 is presented in which data concerning a particular medication, in this case morphine 486, is presented. This data set has already been prepared through use of a suitably functional drug editor, such as that discussed in conjunction with FIG. 21. Infusion delivery limitations are assigned 488. In this case, limitations on PCA dose, loading dose, bolus dose, and a continuous dose have been entered. A concentration limit is specified 492 along with a maximum dose and a minimum accumulated dose 494. The PCA dose limit 496 is specified along with a lockout interval minimum and maximum 498. A "lockout" is the period within which the patient will not be allowed further PCA. Other doses, such as continuous dose 502, loading dose 504, and bolus dose 506 have been specified for this drug. It should be noted that the maximum accumulated dose range is a "soft" limit 508 for this data set. Another data set may include "hard" limits. "Hard" and "soft" limits have been discussed above. A clinical advisory 512 has been assigned to this medication. In certain pumps, interface units, and other processing and/or monitoring devices, clinical advisories are displayed on a monitor screen for the reference of the clinician who is programming the medical device to administer the medication to the patient. Referring to FIG. 2 as an example, when a clinician programs the PCA pump 172 at the central interface unit 100, the clinician will identify the medication contained in the syringe 176. Upon the clinician selecting "morphine" as that medication, the interface unit will present on the display 102 the clinical advisory to "*Continuously monitor respiratory and cardiac function during infusion.*" Such clinical advisories and other data may be added to, edited, or removed from the data set for each medication entry in the drug library through use of the Add Drug, Edit Drug, and Remove Drug keys 514.

Such a drug library created through the editing program discussed above may be transferred to and stored in the memory 250 shown in FIG. 4. The processor controller 264 is programmed to access the drug library during the programming of any infusion device over which it has control. The clinician programming for administration of a medication is required to identify the patient and the medication for infusion. An example would be the case where the clinician is programming the interface unit 100 for the PCA pump 172 located at the left side of the interface unit. The controller would access the drug library contained within itself, or contained within the PCA pump, or contained at a hospital server, on a nurse's station computer, on a PDA, or at another location, compare the patient-specific parameters, such as the ETCO₂ measured by the ETC02 device 94, and permit the programming of the PCA pump or require changes in the programming, as the case may be.

Once programming has occurred and medication administration has begun, the processor controller 264 will continue to monitor the patient's 44 physiological data being measured by the ETCO₂ unit 94, the SpO₂ unit 302 (FIG. 12), or other monitoring unit such as a blood pressure device, temperature device, or other. Based on that physiological data, the controller may automatically alter the programming of the PCA pump 172 to limit the patient's ability to self administer medication. Such alteration may include locking the PCA pump from any further administration of medication to the patient. Another alteration may include a longer time period between boluses of medication from the PCA pump.

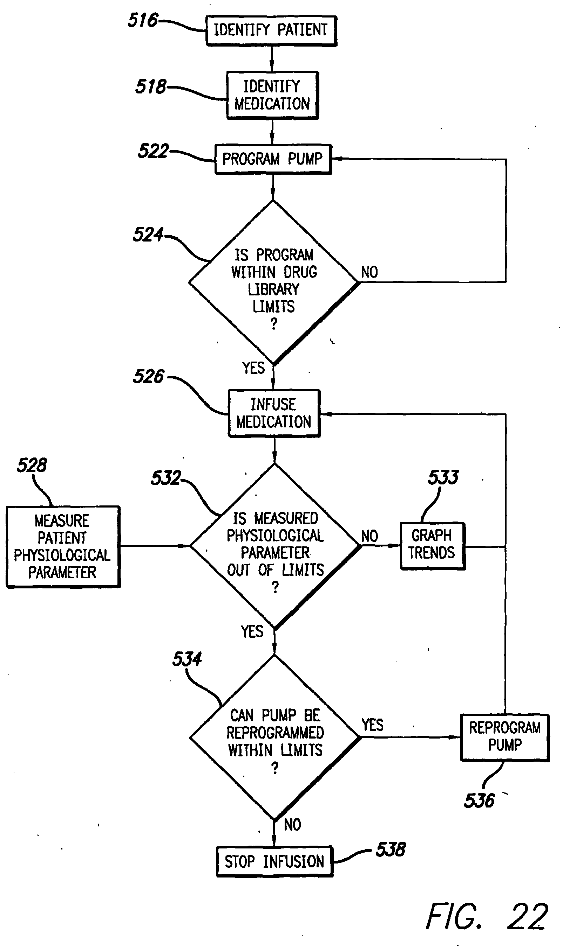

Turning now to FIG. 22, a flow chart of the above method is presented. The patient is identified 516 and then the medication is identified 518 to the processor controller. Identification of the patient can include various details about the patient such as age, weight, allergies, and other data. The processor controller then compares the details of the patient to the drug library for the medication identified. The pump is then programmed 522 and the processor controller verifies 524 that the programming of the pump is within the drug library limits, taking into consideration also the patient data, if any is available, and including any patient physiological monitoring data, if available. If the programming is not within limits, the clinician is requested to confirm that he or she intends to exceed a "soft" limit. If the clinician verifies the intended programming outside the soft limit, the programming will be considered to be within limits. If the programming exceeded a "hard" limit, the clinician will be instructed to reprogram the pump. When the programming is within limits the medication can be infused 526.

During infusion, the processor monitors any measured patient physiological data 528. The processor compares the patient physiological data 532 and if the programming remains within limits considering the physiological data, trends may be graphed 533 in accordance with FIGS. 17 through 20 and infusion continues 526. Other trends may also be graphed. However, if the processor determines that the physiological data indicates that the programming is now outside the limits of the drug library, the processor then determines 534 if the pump can be reprogrammed to be within the drug library limits such as by increasing the lockout period for the PCA pump. If so, the pump is reprogrammed 536 and infusion continues 526. If the pump cannot be reprogrammed to within the limits of the drug library, the pump is stopped 538 and an alarm is given.

The drug library editor program may be run on a computer, such as a desk or laptop computer, separate from the patient care system. The biotechnical staff of a facility, or the pharmacy staff, if a pharmacy is present, may prepare the drug library to be used in that facility. The drug library editor may also be run on other devices such as a PDA. The health care facility using the drug library editor program typically inputs all data into the drug library used in the medical equipment of its facility, although "starter" data sets may be available. Such starter data sets may include a list of the one-thousand most common medications used in a particular country. Additionally, the data set may include common delivery parameters used in a large majority of health care facilities in the particular country as well as allergy information and other data pertaining to the medications contained in the library.

Another example of a Clinical Advisory that may be included in a data set for a drug is shown in FIG. 23. This advisory would be applicable to a PCA application and also indicates that infusion will not be permitted until the advisory has been satisfied. An SpO₂ unit or an ETCO₂ unit must be attached before infusion can begin. This increases safety for the patient in that a physiological parameter of the patient must be monitored before infusion can be started.

Further features include titrating a drug with an infusion pump based on ETCO₂ values, administering a drug reversal agent based on ETCO2 values, restarting an infusion based on improved ETCO₂ values, and increasing a patient lockout period based on ETCO₂ values. Additionally, the controller may store all pump events, such as patient request signals, pump operation parameters, and all measured physiological values of the patient monitor or monitors and provide the stored signals for later analysis. Control over the patient request for further medication delivery can also considered in view of other physiological measurement devices, including a blood pressure monitor, an ECG monitor, a thermometer, and others. Not only can PCA pumps be controlled by such physiological monitoring, but also large volume pumps and other fluid administration devices can be controlled. Further, as discussed above, input from other patient data sources, such as laboratory test results, allergy tests, and emergency medical records, can be considered by the controller in disabling or enabling the patient PCA request device for the patient to self administer medication. Such other information can be obtained from other facility information systems through wired or wireless connection. In the case of problems, alerts may be generated at the medication administration modules themselves, as discussed above, but alerts may also be generated remotely through wired or wireless connection.

Thus there has been provided a PCA system in which patient physiological monitoring is used to control the PCA pump. In certain embodiments, the system includes a drug library with which patient physiological data is compared to determine what, if any, alterations should be made to the PCA pump delivery parameters. Comprehensive drug libraries may be used that include patient-specific considerations in determining if any action is necessary depending on the particular patient performing the PCA. Further, displays can be presented of trends in PCA delivery with physiological data to more easily see the effects or lack of effects of PCA on patient physiological parameters over selectable periods of time.

Although SpO₂ has been used herein in referring to blood-oxygen saturation, this is used as an example or embodiment only. Other devices or methods for the measurement of blood-oxygen saturation may exist or may be developed that will function well. Likewise, ETCO₂ has been used herein also to refer to the level of carbon dioxide. Other devices or techniques for the measurement of this patient physiological parameter may also exist or may be developed in the future.

Although various embodiments of the invention have been described and illustrated, the descriptions are intended to be merely illustrative. It will probably be apparent to those skilled in the art that modifications may be made to the embodiments as described without departing from the scope of the invention as defined by the appended claims.

## Claims

1. A patient care system (300), comprising:
a medication delivery device (172) configured to deliver medication to a patient;
a patient request device (180) with which a patient provides a request signal for delivery of medication;
a physiological monitor (94, 302; 402) disposed to measure a physiological parameter of the patient and provide a physiological signal indicating a value of the measured physiological parameter; and
a controller (100) that receives the request signal and the physiological signal and controls the operation of the medication delivery device (172) to deliver medication to the patient in accordance with the request signal and the physiological signal; wherein the patient care system comprises a display (102) on which the controller (100) presents a trend of the values of the measured physiological parameter (464, 458) with an indication of the point of delivery of the medication (466, 458), wherein the trend comprises a display in graphical form of the values of the measured physiological parameter over a time period **characterised in that** the point of delivery of medication (460) is superimposed on the trend.

2. The patient care system of claim 1 wherein the trend comprises a display in tabular form of the values of the measured physiological parameter over a time period with the point of delivery of medication superimposed on the trend.

3. The patient care system of claim 1 wherein the time period over which the trend is displayed is selectable.

4. The patient care system of claim 1 wherein: the physiological monitor (94; 402) measures ETCO₂ of the patient and provides an ETCO₂ signal; and the controller (100) controls the delivery of medication to the patient based on the ETCO₂ signal.

5. The patient care system of claim 1 wherein: the physiological monitor (302; 402) measures SpO₂ of the patient and provides an SpO₂ signal; and the controller (100) controls the delivery of medication to the patient based on the SpO₂ signal.

6. The patient care system of claim 5 wherein: the physiological monitor (94; 402) measures ETCO₂ of the patient and provides an ETCO₂ signal; and the controller (100) controls the delivery of medication to the patient based on both the SpO₂ signal and on the ETCO₂ signal.

7. The patient care system of claim 4 wherein a waveform of the measured ETCO₂ is displayed.

8. The patient care system of claim 5 wherein a waveform of the measured SpO₂ is displayed.

9. The patient care system of claim 1 wherein in response to the physiological signal the controller (100) controls the operation of the delivery device (172) in accordance with actions consisting of: stop medication delivery; pause medication delivery; disable response to the request signal; establish a time period within which response to the request signal is disabled; and restart medication delivery.

10. The patient care system of claim 1 wherein: the medication delivery device comprises a PCA pump (172) controlled by a patient request to deliver a bolus of medication.

11. The patient care system of claim 10 further comprising a drug library in which is stored data related to analgesic delivery parameters and patient physiological parameters; wherein the controller (100) is programmed to compare the medication delivery request signal and the physiological signal to the stored data of the drug library.

12. The patient care system of claim 4 wherein the controller (100) responds to an ETCO₂ measured value to control the pump to titrate a drug to the patient.

13. The patient care system of claim 4 wherein the controller (100) responds to an ETCO₂ measured value to control the pump to administer a drug reversal agent.

14. The patient care system of claim 4 wherein the controller (100) sends an alert based on an ETCO₂ measured value.

15. The patient care system of claim 12 further comprising a memory in which the controller (100) stores: measured physiological parameter values; and request signals; wherein the controller forwards the stored signals from memory to another location for analysis.

16. The patient care system of claim 1 further comprising a drug library in which ranges of acceptable values for delivery of medication are stored, the controller (100) comparing pumping delivery parameters programmed into the medication delivery device to the drug library and if outside a range of acceptable values, providing an alert.

17. The patient care system of claim 16 wherein:
the drug library includes a soft range of acceptable values, wherein the controller (100) will provide an alert if a programmed parameter of the medication delivery device is outside the soft range however will permit medication delivery to commence as requested;
the drug library includes a hard range of acceptable values, wherein the controller (100) will provide an alert if a programmed parameter of the medication delivery device is outside the hard range and will not permit medication delivery to commence and will require reprogramming of the alerted delivery parameter.

18. The patient care system of claim 15 wherein the controller (100) accesses a data source for patient-specific information and compares it to drug library, provides alert if this patient combined with this drug are outside ranges.

19. The patient care system of claim 15 wherein the data set is configurable by an operator of an editor program.

## Patentansprüche

1. Patientenversorgungssystem (300), das folgendes umfasst:
eine Arzneimittelzufuhrvorrichtung (172), die so gestaltet ist, dass sie einem Patienten Arzneimittel zuführt;
eine Patientenanforderungsvorrichtung (180), mit welcher ein Patient ein Anforderungssignal für die Zufuhr eines Arzneimittels bereitstellt;
einen physiologischen Monitor (94, 302; 402), der so angeordnet ist, dass er einen physiologischen Parameter des Patienten misst und ein physiologisches Signal bereitstellt, das einen Wert des gemessenen physiologischen Parameters anzeigt; und
eine Steuereinrichtung (100), die das Anforderungssignal und das physiologische Signal empfängt und den Betrieb der Arzneimittelzufuhrvorrichtung (172) steuert, so dass dem Patienten ein Arzneimittel gemäß dem Anforderungssignal und dem physiologischen Signal zugeführt wird;
wobei das das Patientenversorgungssystem eine Anzeige (102) umfasst, auf welcher die Steuereinrichtung (100) einen Trend der Werte des gemessenen physiologischen Parameters (464, 458) mit einer Indikation der Zufuhrstelle des Arzneimittels (466, 458) darstellt, wobei der Trend eine Anzeige der Werte des gemessenen physiologischen Parameters über einen Zeitraum in grafischer Form umfasst, **dadurch gekennzeichnet, dass**
die Zufuhrstelle des Arzneimittels (460) den Trend überlagert.

2. Patientenversorgungssystem nach Anspruch 1, wobei der Trend eine Anzeige der Werte des gemessenen physiologischen Parameters über einen Zeitraum in tabellarischer Form umfasst, wobei die Zufuhrstelle des Arzneimittels den Trend überlagert.

3. Patientenversorgungssystem nach Anspruch 1, wobei der Zeitraum, über den der Trend angezeigt wird, wählbar ist.

4. Patientenversorgungssystem nach Anspruch 1, wobei der physiologische Monitor (94; 402) die etCO₂ des Patienten misst und ein etCO₂-Signal bereitstellt; und wobei die Steuereinrichtung (100) die Arzneimittelzufuhr an den Patienten auf der Basis des etCO₂-Signals steuert.

5. Patientenversorgungssystem nach Anspruch 1, wobei: der physiologische Monitor (302; 402) die SpO₂ des Patienten misst und ein SpO₂-Signal bereitstellt; und wobei die Steuereinrichtung (100) die Arzneimittelzufuhr an den Patienten auf der Basis des SpO₂-Signals steuert.

6. Patientenversorgungssystem nach Anspruch 5, wobei: der physiologische Monitor (94; 402) das etCO₂ des Patienten misst und ein etCO₂-Signal bereitstellt; und wobei die Steuereinrichtung (100) die Arzneimittelzufuhr an den Patienten auf der Basis des SpO₂-Signals und des etCO₂-Signals steuert.

7. Patientenversorgungssystem nach Anspruch 4, wobei eine Kurvenform des gemessenen etCO₂ angezeigt wird.

8. Patientenversorgungssystem nach Anspruch 4, wobei eine Kurvenform des gemessenen SpO₂ angezeigt wird.

9. Patientenversorgungssystem nach Anspruch 1, wobei die Steuereinrichtung (100) als Reaktion auf das physiologische Signal den Betrieb der Zufuhrvorrichtung (172) gemäß Aktionen steuert, welche folgende umfassen: Anhalten der Arzneimittelzufuhr; Unterbrechung der Arzneimittelzufuhr; Deaktivierung der Reaktion auf das Anforderungssignal; Festlegung eines Zeitraums, innerhalb welchem die Reaktion auf das Anforderungssignal deaktiviert ist; und Wiederaufnahme der Arzneimittelzufuhr.

10. Patientenversorgungssystem nach Anspruch 1, wobei die Arzneimittelzufuhrvorrichtung eine PCA-Pumpe (172) umfasst, die durch eine Patientenanforderung für die Zufuhr eines Bolus eines Arzneimittels gesteuert wird.

11. Patientenversorgungssystem nach Anspruch 10, wobei dieses ferner eine Arzneimittelbibliothek umfasst, in der Daten gespeichert sind, die sich auf analgetische Zufuhrparameter und physiologische Patientenparameter beziehen; wobei die Steuereinrichtung (100) so programmiert ist, dass sie das Anforderungssignal für eine Arzneimittelzufuhr und das physiologische Signal mit den gespeicherten Daten der Arzneimittelbibliothek vergleicht.

12. Patientenversorgungssystem nach Anspruch 4, wobei die Steuereinrichtung (100) auf einen gemessenen etCO₂-Wert so reagiert, dass die Pumpe so gesteuert wird, dass ein Arzneimittel an den Patienten titriert wird.

13. Patientenversorgungssystem nach Anspruch 4, wobei die Steuereinrichtung (100) auf einen gemessenen etCO₂-Wert so reagiert, dass die Pumpe so gesteuert wird, dass ein Arzneimittel-Antidot verabreicht wird.

14. Patientenversorgungssystem nach Anspruch 4, wobei die Steuereinrichtung (100) auf der Basis eines gemessenen etCO₂-Wertes eine Warnmeldung sendet.

15. Patientenversorgungssystem nach Anspruch 12, wobei dieses ferner einen Speicher umfasst, in dem die Steuereinrichtung (100) folgendes speichert: gemessene physiologische Parameterwerte und Anforderungssignale; wobei die Steuereinrichtung die gespeicherten Signale von dem Speicher an eine andere Stelle zur Analyse weiterleitet.

16. Patientenversorgungssystem nach Anspruch 10, wobei dieses ferner eine Arzneimittelbibliothek umfasst, in welcher Bereiche zulässiger Werte für die Arzneimittelzufuhr gespeichert sind, wobei die Steuereinrichtung (100) die in der Arzneimittelzufuhrvorrichtung programmierten Pumpzufuhrparameter mit der Arzneimittelbibliothek vergleicht, und wobei sie eine Warnmeldung bereitstellt, wenn die Parameter außerhalb eines Bereichs zulässiger Werte liegen.

17. Patientenversorgungssystem nach Anspruch 16, wobei:
die Arzneimittelbibliothek einen weichen Bereich zulässiger Werte aufweist, wobei die Steuereinrichtung (100) eine Warnmeldung bereitstellt, wenn ein programmierter Parameter der Arzneimittelzufuhrvorrichtung außerhalb des weichen Bereichs liegt, wobei sie jedoch das Einsetzen der Arzneimittelzufuhr wie angefordert zulässt;
die Arzneimittelbibliothek einen harten Bereich zulässiger Werte aufweist, wobei die Steuereinrichtung (100) eine Warnmeldung bereitstellt, wenn ein programmierter Parameter der Arzneimittelzufuhrvorrichtung außerhalb des harten Bereichs liegt, und wobei sie das Einsetzen der Arzneimittelzufuhr nicht zulässt und eine neue Programmierung des Zufuhrparameters erfordert, für den die Warnmeldung bereitgestellt worden ist.

18. Patientenversorgungssystem nach Anspruch 15, wobei die Steuereinrichtung (100) auf eine Datenquelle für patientenspezifische Informationen zugreift und diese mit der Arzneimittelbibliothek vergleicht und eine Warnmeldung bereitstellt, wenn dieser Patient in Verbindung mit dem Arzneimittel außerhalb der Bereiche liegt.

19. Patientenversorgungssystem nach Anspruch 154, wobei die Datenmenge durch eine Bedienungsperson eines Bearbeitungsprogramms konfigurierbar ist.

## Revendications

1. Système de soins pour patients (300), comprenant :
un dispositif d'administration de médicament (172) conçu pour administrer un médicament à un patient ;
un dispositif de demande de patient (180) par lequel un patient émet un signal de demande pour l'administration de médicament ;
un moniteur physiologique (94, 302 ; 402) disposé pour mesurer un paramètre physiologique du patient et émettre un signal physiologique indquant une valeur du paramètre physiologique mesuré ; et
un dispositif de commande (100) qui reçoit le signal de demande et le signal physiologique et commande le fonctionnement du dispositif d'administration de médicament (172) pour administrer un médicament au patient conformément au signal de demande et au signal physiologique ;
le système de soins pour patients comprenant un affichage (102) sur lequel le dispositif de commande (100) présente une tendance des valeurs du paramètre physiologique mesuré (464, 458) avec une indication du point d'administration du médicament (466, 458), la tendance comprenant un affichage sous forme graphique des valeurs du paramètre physiologique mesuré sur une période de temps **caractérisée en ce que** le point d'administration de médicament (460) est superposé sur la tendance.

2. Système de soins pour patients selon la revendication 1, la tendance comprenant un affichage sous forme de tableau des valeurs du paramètre physiologique mesuré sur une période de temps, le point d'administration de médicament étant superposé sur la tendance.

3. Système de soins pour patients selon la revendication 1, la période de temps sur laquelle la tendance est affichée étant sélectionnable.

4. Système de soins pour patients selon la revendication 1, le moniteur physiologique (94 ; 402) mesurant l'ETCO₂ du patient et émettant un signal ETCO₂ ; et le dispositif de commande (100) commandant l'administration de médicament au patient en fonction du signal ERCO₂.

5. Système de soins pour patients selon la revendication 1, le moniteur physiologique (302 ; 402) mesurant le SpO₂ du patient et émettant un signal SpO₂ ; et le dispositif de commande (100) commandant l'administration de médicament au patient en fonction du signal SpO₂.

6. Système de soins pour patients selon la revendication 5, le moniteur physiologique (94 ; 402) mesurant l'ETCO₂ du patient et émettant un signal ETCO₂ ; et le dispositif de commande (100) commandant l'administration de médicament au patient en fonction du signal SpO₂ et du signal ERCO₂.

7. Système de soins pour patients selon la revendication 4, affichant une forme d'onde de l'ETCO₂.

8. Système de soins pour patients selon la revendication 5, affichant une forme d'onde du SpO₂ mesuré.

9. Système de soins pour patients selon la revendication 1, en réponse au signal physiologique, le dispositif de commande (100) commandant le fonctionnement du dispositif d'administration (172) conformément aux actions consistant à : arrêter l'administration de médicament ; mettre en pause l'administration de médicament ; désactiver la réponse au signal de demande ; établir une période de temps au cours de laquelle la réponse au signal de demande est désactivée ; et redémarrer l'administration de médicament.

10. Système de soins pour patients selon la revendication 1, le dispositif d'administration de médicament comprenant une pompe PCA (172) commandée par une demande de patient d'administration d'un bol intraveineux de médicament.

11. Système de soins pour patients selon la revendication 10, comprenant en outre une bibliothèque de médicaments dans laquelle sont stockées des données concernant des paramètres d'administration d'analgésique et des paramètres physiologiques de patient ; le dispositif de commande (100) étant programmé pour comparer le signal de demande d'administration de médicament et le signal physiologique aux données stockées de la bibliothèque de médicaments.

12. Système de soins pour patients selon la revendication 4, le dispositif de commande (100) répondant à une valeur mesurée ETCO₂ pour commander la pompe pour titrer un médicament au patient.

13. Système de soins pour patients selon la revendication 4, le dispositif de commande (100) répondant à une valeur mesurée ETCO₂ pour commander la pompe pour administrer un agent d'inversion de médicament.

14. Système de soins pour patients selon la revendication 4, le dispositif de commande (100) envoyant une alerte basée sur une valeur mesurée ETCO₂.

15. Système de soins pour patients selon la revendication 12, comprenant en outre une mémoire dans laquelle le dispositif de commande (100) stocke : des valeurs de paramètre physiologique mesuré ; et des signaux de demande ; le dispositif de commande transmettant les signaux stockés de la mémoire à un autre emplacement pour analyse.

16. Système de soins pour patients selon la revendication 1, comprenant en outre une bibliothèque de médicaments dans laquelle sont stockées les plages de valeurs acceptables pour l'administration de médicament, le dispositif de commande (100) comparant les paramètres d'administration de pompage programmés dans le dispositif d'administration de médicament à la bibliothèque de médicaments et, s'ils sont hors d'une plage de valeurs acceptables, émettant une alerte.

17. Système de soins pour patients selon la revendication 16,
la bibliothèque de médicaments comprenant une plage souple de valeurs acceptables, le dispositif de commande (100) émettant une alerte si un paramètre programmé du dispositif d'administration de médicament est en hors de la plage souple mais permettant toutefois à l'administration de médicament de commencer comme demandé ;
la bibliothèque de médicaments comprenant une plage fixe de valeurs acceptables, le dispositif de commande (100) émettant une alerte si un paramètre programmé du dispositif d'administration de médicament est hors de la plage fixe et ne permettant pas à l'administration de médicament de commencer et exigeant une reprogrammation du paramètre d'administration objet de l'alerte.

18. Système de soins pour patients selon la revendication 15, le dispositif de commande (100) accédant à une source de données pour des informations spécifiques au patient et les comparant à la bibliothèque de médicaments, émettant une alerte si ce patient combiné à ce médicament est en hors des plages.

19. Système de soins pour patients selon la revendication 15, l'ensemble de données étant configurable par un opérateur d'un programme d'éditeur.
